(19) Europäisches Patentamt
European Patent Office
Office européen des brevets

(11) **EP 1 531 734 B1**

(12) **FASCICULE DE BREVET EUROPEEN**

(45) Date de publication et mention de la délivrance du brevet:
**02.07.2014 Bulletin 2014/27**

(21) Numéro de dépôt: **03758254.1**

(22) Date de dépôt: **20.08.2003**

(51) Int Cl.:
*A61B 8/00* *(2006.01)* *A61B 8/08* *(2006.01)*
*A61N 7/02* *(2006.01)* *A61B 5/107* *(2006.01)*
*G01S 7/52* *(2006.01)* *A61B 5/00* *(2006.01)*

(86) Numéro de dépôt international:
**PCT/FR2003/002554**

(87) Numéro de publication internationale:
**WO 2004/019784 (11.03.2004 Gazette 2004/11)**

(54) **PROCEDE NON INVASIF POUR OBTENIR UN CHAMP PREDETERMINE D'ONDES ACOUSTIQUES DANS UN MILIEU SENSIBLEMENT HOMOGENE MASQUE PAR UNE BARRIERE OSSEUSE, PROCEDE D'IMAGERIE ET DISPOSITIF POUR LA MISE EN OEUVRE DE CES PROCEDES**

NICHTINVASIVES VERFAHREN ZUR ERZEUGUNG EINES VORBESTIMMTEN FELDES AKUSTISCHER WELLEN IN EINER VON EINER KNOCHENBARRIERE VERBORGENEN IM WESENTLICHEN HOMOGENEN UMGEBUNG, ABBILDUNGSVERFAHREN UND VORRICHTUNG ZUR DURCHFÜHRUNG DIESES VERFAHRENS

NON-INVASIVE METHOD OF OBTAINING A PRE-DETERMINED ACOUSTIC WAVE FIELD IN AN ESSENTIALLY UNIFORM MEDIUM WHICH IS CONCEALED BY A BONE BARRIER, IMAGING METHOD AND DEVICE FOR CARRYING OUT SAID METHODS

(84) Etats contractants désignés:
**AT BE BG CH CY CZ DE DK EE ES FI FR GB GR HU IE IT LI LU MC NL PT RO SE SI SK TR**

(30) Priorité: **28.08.2002 FR 0210682**

(43) Date de publication de la demande:
**25.05.2005 Bulletin 2005/21**

(73) Titulaire: **SUPER SONIC IMAGINE**
**13857 Aix-en-Provence (FR)**

(72) Inventeurs:
• **AUBRY, Jean-François**
**F-92340 Bourg la Reine (FR)**

• **FINK, Mathias**
**F-92190 Meudon (FR)**
• **TANTER, Mickael**
**F-75006 Paris (FR)**

(74) Mandataire: **Cabinet Plasseraud**
**52, rue de la Victoire**
**75440 Paris Cedex 09 (FR)**

(56) Documents cités:
**WO-A1-02/32316    FR-A- 2 791 136**
**FR-A- 2 815 717    US-A- 5 010 885**
**US-A- 5 276 654**

**Description**

La présente invention est relative aux procédés non invasifs pour obtenir un champ prédéterminé d'ondes acoustiques dans un milieu sensiblement homogène masqué par une barrière osseuse, aux, procédés d'imagerie médicale mettant en oeuvre ces procédés, et aux dispositifs pour la mise en oeuvre de ces procédés.

Plus particulièrement, l'invention concerne un procédé non invasif pour obtenir au moins un champ objectif prédéterminé d'ondes acoustiques dans un milieu sensiblement homogène masqué (totalement ou partiellement) par une barrière osseuse, en faisant émettre des signaux acoustiques par au moins un réseau de transducteurs (comprenant un ou plusieurs transducteurs, et pouvant comprendre plusieurs sous-réseaux de transducteurs) à travers ladite barrière osseuse.

On entend ici par milieu sensiblement homogène un milieu ayant des caractéristiques sensiblement homogènes pour la propagation des ondes acoustiques. Un tel milieu sensiblement homogène peut par exemple être constitué par le cerveau, auquel cas la barrière osseuse est constituée par le crâne. Le cas échéant, milieu sensiblement homogène pourrait être constitué par le coeur ou l'ensemble coeur-poumons, auquel cas la barrière osseuse serait constituée par la cage thoracique.

On notera que le champ objectif d'ondes acoustiques en question peut consister en une onde impulsionnelle focalisée en un ou plusieurs points du cerveau, ou en un champ spatio-temporel plus complexe.

Le document WO-A-02/32316 décrit un exemple d'un tel procédé, qui donne toute satisfaction au plan de ses résultats.

La présente invention a notamment pour but de perfectionner encore ce type de procédé, notamment afin d'obtenir une focalisation plus précise des ondes acoustiques.

A cet effet, selon l'invention, un procédé du genre en question est caractérisé en ce qu'il comporte :

- une phase d'apprentissage comprenant au moins les étapes suivantes :

   (1a) réaliser, au moins partiellement par rayons X, une image tridimensionnelle de la barrière osseuse, donnant un paramètre représentatif de la porosité de ladite barrière osseuse en différents points,
   (1b) à partir de ladite image tridimensionnelle, déterminer au moins des cartographies tridimensionnelles de masse volumique, de célérité des ondes acoustiques et d'absorption des ondes acoustiques dans ladite barrière osseuse,
   (1c) déterminer un positionnement prévu du réseau de transducteurs par rapport à la barrière osseuse,
   (1d) simuler au moins une propagation d'ondes acoustiques entre au moins un point du milieu sensiblement homogène et au moins certains transducteurs du réseau de transducteurs (dans au moins un sens, du milieu sensiblement homogène vers le réseau de transducteurs, et/ou inversement), à partir d'un modèle mathématique de propagation et desdites cartographies tridimensionnelles de masse volumique, de célérité des ondes acoustiques et d'absorption des ondes acoustiques,
   (1e) à partir de ladite simulation, calculer des signaux acoustiques élémentaires à émettre par au moins certains transducteurs dudit réseau de transducteurs pour obtenir ledit champ objectif d'ondes acoustiques,

- et une phase de positionnement réel du réseau de transducteurs sur la barrière osseuse, comprenant les étapes suivantes :

   (2a) positionner, d'abord approximativement le réseau de transducteurs sur la.barrière osseuse, dans sa position prévue,
   (2b) faire repérer par au moins certains transducteurs du réseau de transducteurs, par échographie, une positon relative entre ledit réseau de transducteurs et là barrière osseuse,
   (2c) et affiner la position du réseau de transducteurs par rapport à ladite barrière osseuse en fonction du repérage effectué à l'étape (2b), de façon'que ladite position relative corresponde au positionnement prévu.

Grâce à ces dispositions, on obtient des signaux acoustiques élémentaires qu'un praticien peut ensuite utiliser en fonction de ses besoins pour obtenir une focalisation très précise d'ondes acoustiques réelles en des zones du milieu sensiblement homogène qu'il détermine (ou plus généralement, pour générer très précisément un champ d'ondes souhaité), par exemple aux fins d'imagerie médicale statique ou fonctionnelle et/ou aux fins de traitement par hyperthermie.

Dans des modes de réalisation préférés du procédé selon l'invention, on peut éventuellement avoir recours en outre à l'une et/ou à l'autre des dispositions suivantes :

- au cours de l'étape (2b), on repère la position relative entre ledit réseau de transducteurs et la barrière osseuse, en déterminant une forme extérieure d'au moins une partie de la barrière osseuse par échographie, par au moins une partie dudit réseau de transducteurs, en comparant cette forme extérieure à ladite image tridimensionnelle de la barrière osseuse ;

- l'étape (1a) est précédée d'une étape initiale au cours de laquelle on fixe rigidement un dispositif de repérage sur ladite barrière osseuse, et ledit dispositif de repérage étant adapté pour absorber (au moins partiellement) des rayons X,
 au cours de l'étape (1a), l'image tridimensionnelle réalisée donne également un positionnement du dispositif de repérage sur la barrière osseuse,
 et au cours de l'étape (2b), on repère par échographie une position relative entre le réseau de transducteurs et le dispositif de positionnement ;

- le réseau de transducteurs est inclus dans un réservoir rempli de produit fluide (liquide, gel ou similaire) et comprenant au moins une paroi souple, et au cours de la phase de positionnement, cette paroi souple est placée en appui contre la barrière osseuse ;

- le milieu sensiblement homogène comprend au moins une partie d'un cerveau et la barrière osseuse comprend au moins une partie d'un crâne entourant ce cerveau ;

- les ondes acoustiques ont des fréquences comprises entre 0,5 et 3 MHz ;

- l'étape (1e) est suivie d'une étape (1f) au cours de laquelle on simule au moins une émission, par le réseau de transducteurs, de signaux acoustiques déterminés à partir desdits signaux acoustiques élémentaires et permettant d'obtenir un champ d'ondes acoustiques souhaité, on simule une propagation d'ondes acoustiques générées par cette émission, et on vérifie que cette propagation satisfait à certains critères prédéfinis. (notamment, qualité de la focalisation, plage de température à atteindre localement par l'échauffement dû aux ondes acoustiques, absence de cavitation, etc.) ;

- au cours de l'étape (1d), on simule une propagation d'ondes acoustiques depuis au moins un point du milieu sensiblement, homogène vers au moins certains transducteurs du réseau de transducteurs (notamment en simulant l'émission d'une impulsion d'onde acoustique audit point du milieu sensiblement homogène) et on détermine des signaux acoustiques simulés reçus Ri(t) arrivant à l'emplacement desdits transducteurs i du réseau de transducteurs, i étant un entier compris entre 1 et n et n étant le nombre de transducteurs du réseau de transducteurs, et au cours de l'étape (1e), on détermine les signaux acoustiques élémentaires Ei(t), à émettre par chaque transducteur i considéré, comme étant proportionnels à une inversion temporelle Ri(-t) desdits signaux acoustiques simulés reçus Ri(t), précédemment déterminés à l'étape (1d) ;

- au cours de l'étape (1e), on détermine les signaux acoustiques à émettre Ei(t) par la formule : Ei(t)=Gi. Ri(-t), où Gi est un facteur de gain différent d'un transducteur i à l'autre, pour compenser les dissipations dans la barrière osseuse ;

- les gains Gi correspondant à au moins certaines transducteurs sont respectivement inversement proportionnels au carré d'une amplitude des signaux acoustiques simulés reçus Ri(t) correspondants ;

- au cours de l'étape (1d), on effectue la simulation en utilisant une cartographie tridimensionnelle fictive d'absorption des ondes acoustiques, ayant en chaque point de la barrière osseuse des coefficients d'absorption $-\tau$ opposés à des coefficients d'absorption réels $\tau$ déterminés au cours de l'étape (1b), et au cours de l'étape (1e), on détermine les signaux acoustiques élémentaires à émettre Ei(t) comme étant égaux à ladite inversion, temporelle Ri (-t) ;

- le réseau de transducteurs est inclus dans un réservoir rempli de produit fluide (liquide, gel ou similaire) et comprenant au moins une paroi souple destinée à être placée en appui contre la barrière osseuse, les emplacements prévus des transducteurs, déterminés au cours de l'étape (1c), n'étant pas au contact de la barrière osseuse,

au cours de l'étape (1d) :

. on simule une propagation d'ondes acoustiques depuis au moins un point du milieu sensiblement homogène vers au moins certains transducteurs du réseau de transducteurs (notamment en simulant l'émission d'une impulsion d'onde acoustique audit point du milieu sensiblement homogène) et on détermine des signaux acoustiques simulés reçus Ri(t) arrivant à l'emplacement desdits transducteurs i du réseau de transducteurs, i étant un entier compris entre 1 et n et n étant le nombre de transducteurs du réseau de transducteurs,

puis on simule une émission par chaque transducteur i d'un signal acoustique Ri(-t) correspondant à une inversion temporelle' du signal Ri(t), et une propagation dans ledit produit fluide jusqu'à un transducteur fictif i situé au contact de la barrière osseuse en correspondance avec le transducteur i, et on détermine des signaux acoustiques simulés reçus R'i(t) arrivant à l'emplacement dudit transducteur fictif i,

. puis on simule une émission par chaque transducteur fictif i d'un signal acoustique G'i.R'i(-t) où R'i(-t) est une inversion temporelle du signal R'i(t) et G'i est un coefficient inversement proportionnel au carré d'une amplitude du signal R'i(t) au moins pour certains transducteurs fictifs i,

. puis on simule une propagation dans ledit produit fluide jusqu'au transducteur i, et on détermine des signaux acoustiques simulés reçus R''i(t) arrivant à l'emplacement dudit transducteur i,

et au cours de l'étape (1e), on détermine les signaux acoustiques élémentaires à émettre Ei(t) comme étant égaux à une inversion temporelle R"i(-t) desdits signaux acoustiques simulés reçus R''i(t) ;

- au cours de l'étape (1d), on simule l'émission d'une impulsion d'ondes acoustiques par au moins certains transducteurs i du réseau de transducteurs - et une propagation d'ondes acoustiques depuis chaque transducteur i considéré vers plusieurs points de référence r situés dans le milieu sensiblement homogène, i étant un indice compris entre 1 et n qui désigne un transducteur du réseau et n étant un entier naturel non nul qui désigne le nombre de transducteurs, r étant un entier compris entre 1 et m et m étant un entier naturel non nul désignant le nombre de points de référence, et on détermine des réponses impulsionnelle simulées hri(t) arrivant en chacun desdits points de référence r du milieu sensiblement homogène, l'étape (1e) comprenant les sous-étapes suivantes :

    (1e1) on détermine un nombre p de composantes fréquentielles de chacune de ces réponses impulsionnelles simulées, de fréquences respectives $\omega k$, k étant un indice compris entre 1 et p qui désigne une composante fréquentielle,
    (1e2) on détermine p matrices de transfert $H(\omega k)=[Hri(\omega k)]$, i allant de 1 à n et r allant de 1 à m, où $Hri(\omega k)$ est la valeur à la fréquence $\omega k$, de la transformée de Fourier de la réponse impulsionnelle Hri(t),
    (1e3) on détermine, pour chaque point de référence r, n composantes $Ei(\omega k,r)$ telles que $F(\omega k,r)=H(\omega k).E(\omega k,r)$, où $E(\omega k,r)=[Ei(\omega k,r)]$ est un vecteur à n composantes $Ei(\omega,r)$, $F(\omega k,r)=[F1(\omega k,r)]$ est un vecteur à m composantes $Fl(\omega k, r)$ où l varie entre 1 et - m, ces m composantes $Fl(\omega k, r)$ correspondant à la génération dudit champ objectif prédéterminé d'ondes acoustiques à la fréquence $\omega k$ aux points r;

- au cours de la sous-étape (1e3), on calcule p matrices $H^{-1}(\omega k)$ au moins par inversion des matrices de transfert de $H(\omega k)$, et pour chaque point de référence r du milieu sensiblement homogène, on calcule le vecteur $E(\omega k, r)$ par la formule :

$$E(\omega k, r) = H^{-1}(\omega k) \cdot F(\omega k, r) \; ;$$

- au cours de l'étape, (1d), on détermine des réponses impulsionnelles hir(t) entre plusieurs points de référence r du milieu sensiblement homogène et au moins certains transducteurs i du réseau de transducteurs, i étant un indice compris entre 1 et n qui désigne un transducteur et n étant un entier naturel non nul qui désigne le nombre de transducteurs, r étant un entier compris entre 1 et m et m étant un entier naturel non nul désignant le nombre de points de référence, et au cours de l'étape (1e), on détermine également comment focaliser' au moins une partie du réseau de transducteur en réception sur chaque point de référence r pour réaliser une image échographique.

Par ailleurs, l'invention a également pour objet un procédé d'imagerie médicale par échographie, comprenant un procédé non invasif pour obtenir un champ objectif d'ondes acoustiques tel que défini ci-dessus, et une phase d'imagerie au cours de laquelle on réalise au moins une image échographique du milieu sensiblement homogène à l'aide d'au moins une partie du réseau de transducteurs, en utilisant les signaux acoustiques élémentaires déterminés au cours de la phase d'apprentissage.
Enfin, l'invention a aussi pour objet un dispositif spécialement conçu pour la mise en oeuvre d'un procédé tel que défini ci-dessus, ce dispositif comprenant au moins :

- un réseau de transducteurs adapté pour être positionné à l'extérieur d'une barrière osseuse masquant un milieu sensiblement homogène,
- des moyens pour déterminer au moins des cartographies tridimensionnelles de masse volumique, de célérité des ondes acoustiques et d'absorption des ondes acoustiques dans la barrière osseuse, à partir d'une image tridimensionnelle de ladite barrière osseuse réalisée par rayons X et donnant la porosité de ladite la barrière osseuse en chaque point,

- des moyens pour simuler au moins une propagation d'ondes acoustiques entre au moins un point du milieu sensiblement homogène et au moins certains transducteurs du réseau de transducteurs, à partir d'un modèle mathématique de propagation et desdites cartographies tridimensionnelles de masse volumique, de célérité des ondes acoustiques et d'absorption des ondes acoustiques, et en fonction d'un positionnement prévu du réseau de transducteurs par rapport à la barrière osseuse,
- des moyens pour calculer, à partir de ladite simulation, des signaux acoustiques élémentaires à émettre par au moins certains transducteurs dudit réseau de transducteurs pour obtenir un champ objectif d'ondes acoustiques dans le milieu sensiblement homogène,

- des moyens pour faire repérer par au moins certains transducteurs du réseau de transducteurs, par échographie, une position relative entre ledit réseau de transducteurs et la barrière osseuse,
- et des moyens pour affiner une position relative initiale du réseau de transducteurs par rapport à la barrière osseuse en fonction du repérage de la position relative entre le réseau de transducteurs et la barrière osseuse, de façon que ladite position relative du réseau de transducteur par rapport à la barrière osseuse corresponde au positionnement prévu.

Dans des modes de réalisation préférés du dispositif selon l'invention, on peut éventuellement avoir recours en outre à l'une et/ou à l'autre des dispositions suivantes :

- les moyens pour repérer la position relative entre ledit réseau de transducteurs et la barrière osseuse sont adaptés pour déterminer une forme extérieure d'au moins une partie de la barrière osseuse par échographie, par l'intermédiaire d'au moins une partie dudit réseau de transducteurs, en comparant cette forme extérieure à ladite image tridimensionnelle de la barrière osseuse ;
- le dispositif d'imagerie comprend un dispositif de repérage doté de moyens de fixation adaptés pour fixer rigidement ledit dispositif de repérage sur la barrière osseuse, et ledit dispositif de repérage étant adapté pour absorber des rayons X et être visible sur l'image tridimensionnelle de ladite barrière osseuse, et les moyens pour repérer la position relative entre ledit réseau de transducteurs et la barrière osseuse sont adaptés pour repérer par échographie une position relative entre le réseau de transducteurs et le dispositif de positionnement ;
- le réseau de transducteurs est inclus dans un réservoir rempli de produit fluide (liquide, gel ou similaire) et comprenant au moins une paroi souple destinée à venir en appui contre la barrière osseuse ;
- le réseau de transducteurs comprend un sous-réseau de traitement par hyperthermie et un sous-réseau d'imagerie, ces deux sous-réseaux comportant respectivement des transducteurs de types différents.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue d'ensemble schématique d'un dispositif de génération d'ondes acoustiques ultrasonores selon une forme de réalisation de l'invention,
- la figure 2 est une vue de détail en coupe partielle d'un casque appartenant au dispositif de la figure 1, installé sur la tête d'un patient,
- et la figure 3 est un schéma fonctionnel du dispositif de la figure 1.

Sur les différentes figurés, les mêmes références désignent des éléments identiques ou similaires.

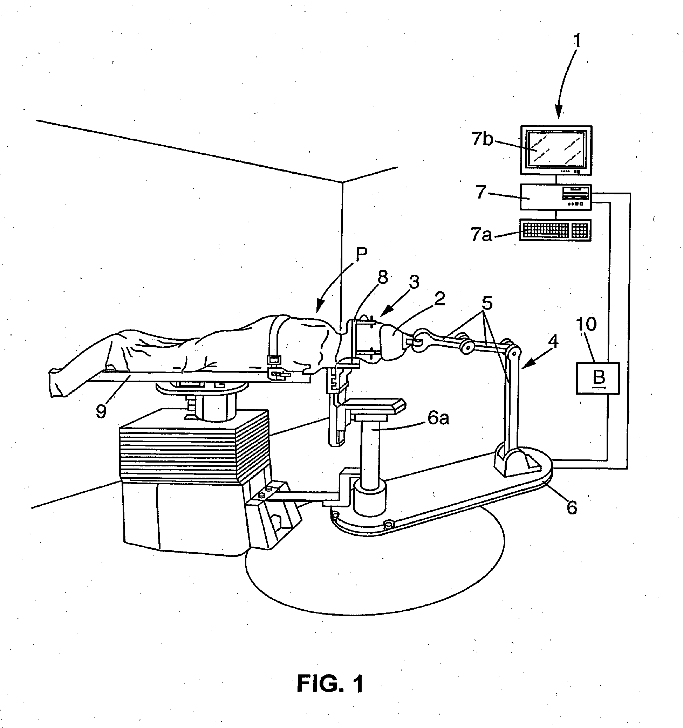

Le dispositif 1 de génération d'ondes acoustiques représenté sur la figure 1 est adapté pour générer des champs d'ondes acoustiques prédéterminés dans un milieu sensiblement homogène du corps d'un patient P, ce milieu sensiblement homogène étant au moins partiellement masqué par une barrière osseuse.

Dans l'exemple visible sur les dessins, le milieu sensiblement homogène est constitué par le cerveau du patient, et la barrière osseuse est constituée par son crâne.

Plus généralement, le milieu sensiblement homogène pourrait être tout milieu tissulaire d'un patient humain ou de tout autre vertébré, milieu tissulaire ayant des caractéristiques sensiblement homogènes pour la propagation des ondes acoustiques. Ce milieu tissulaire pourrait éventuellement être constitué par le coeur ou l'ensemble coeur-poumons du patient P, auquel cas la barrière osseuse serait constituée par la cage thoracique.

Le dispositif 1 est destiné à générer des ondes acoustiques ultrasonores dans le cerveau du patient P, à des fréquences par exemple de l'ordre de 0,5 à 3 MHz, depuis l'extérieur du crâne.

Cette génération d'ondes acoustiques peut être destinée par exemple :

- à réaliser une image ou une série d'images échographiques du cerveau, qu'il s'agisse d'imagerie statique ou fonctionnelle, notamment d'imagerie Doppler permettant de visualiser les écoulements sanguins ou d'imagerie thermique permettant de visualiser les échauffement provoqués par un traitement par hyperthermie,
- et/ou de réaliser 1 un traitement par hyperthermie, notamment pour :
- détruire des tumeurs bénignes ou malignes, simples ou multiples,
- coaguler des hémorragies (repérées par imagerie fonctionnelle Doppler comme indiqué ci-dessus),
- activer localement des médicaments thermo-activables,
- briser localement la barrière hémato-encéphalique afin de faire diffuser localement un médicament précédemment injecté par voie intraveineuse.

Dans tous les cas, il est nécessaire de pouvoir générer, avec le plus de précision possible, un ou plusieurs champs objectifs prédéterminés d'ondes acoustiques dans le cerveau de patient P, par exemple pour focaliser en un ou plusieurs points du cerveau les ondes acoustiques émises par un réseau de transducteurs, ou pour générer des champs d'ondes plus complexes.

Le réseau de transducteurs, non visible sur la figure 1, peut être par exemple intégré dans un casque 2 venant en appui au sommet de la tête 3 du patient P et porté par un bras robotisé 4 (ou tout autre système de positionnement) comprenant plusieurs bras de levier 5 articulés les uns aux autres et portés par une embase 6 fixée au sol. Pour positionner précisément la tête 3 du patient par rapport au référentiel du bras robotisé, on peut éventuellement prévoir un support 6a solidaire de l'embase 6, sur lequel vient se fixer un cadre rigide de stéréotaxie 8 lui-même fixé rigidement sur le crâne du patient P, pendant que le patient P est sur une table 9.

Le bras robotisé 6 ou autre dispositif de positionnement est avantageusement-commande par un micro-ordinateur 7 ou similaire, doté d'au moins une interface d'entrée tel qu'un clavier 7a et d'au moins une interface de sortie tel qu'un écran 7b. Le micro-ordinateur 7 commande par ailleurs le fonctionnement du réseau de transducteurs inclus dans le casque 2, directement ou de préférence par l'intermédiaire d'une baie électronique 10 (B) de traitement de signal.

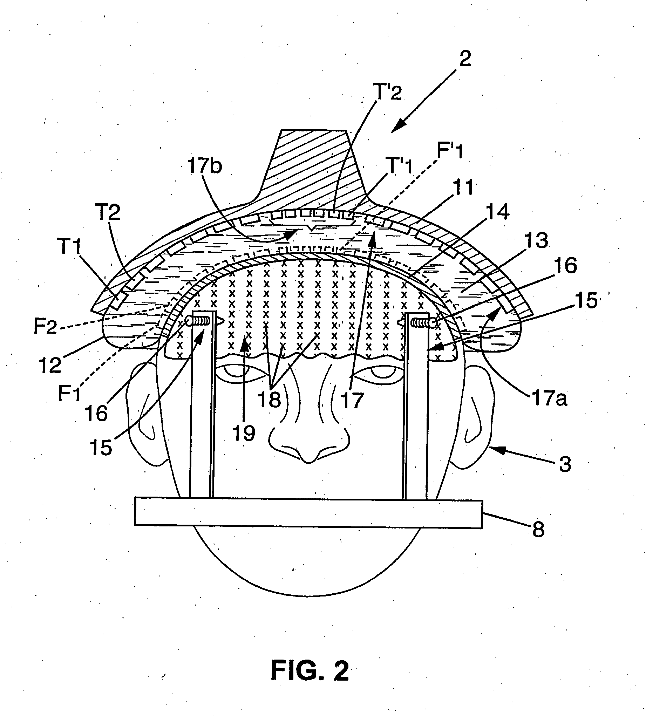

Comme représenté sur la figure 2, le casque 2 peut par exemple comporter une coupole rigide 11 qui délimite, avec une paroi, souple 12, un réservoir 13 rempli d'un produit fluide tel qu'un gel ou un liquide, notamment de l'eau. Le réseau de transducteurs 17 est immergé dans ce produit fluide sur la face intérieure de la coupole 11.

Lorsque le casque 2 est positionné contre le sommet de la tête 3 du patient par le bras robotisé 4, la paroi souple 12 vient épouser exactement la forme du crâne 14 du patient, en recouvrant le cas échéant les extrémités supérieures de bras de fixation rigides 15 du cadre' de stéréotaxie 8, bras qui peuvent être fixés au crâne 14 par des vis 16.

Le réseau 17 de transducteurs peut comprendre un nombre total, n de transducteurs ultrasonores utilisés uniquement aux fins d'imagerie, ou des transducteurs utilisés uniquement aux fins de traitement par hyperthermie, ou encore des transducteurs ultrasonores utilisés à la fois aux fins d'imagerie et de traitement par hyperthermie.

Eventuellement, le réseau 17 de transducteurs peut comprendre deux sous-réseaux :

- un sous-réseau de traitement par hyperthermie 17a, qui comprend un nombre n1 au moins égal à 1, par exemple supérieur à 100, voire supérieur à 200, de transducteurs T1, T2, T3,... Tn1 qui sont en liaison acoustique avec le crâne 14 du patient par l'intermédiaire du produit fluide,
- et un sous-réseau d'imagerie 17b, qui comprend un nombre n2 au moins égal à 1 (avec n1 + n2 = n), par exemple supérieur à 100, voire supérieur à 200, de transducteurs T'1, T'2, T'3,... T'n2 qui sont en liaison acoustique avec le crâne 14 du patient par l'intermédiaire du produit fluide et qui peuvent par exemple être regroupés en une barrette centrale disposée sensiblement dans le plan sagital de la tête 3 du patient.

Les transducteurs des sous-réseaux 17a, 17b sont avantageusement de deux types différents. Les transducteurs du sous-réseau 17a peuvent ainsi être plus grands que les transducteurs du sous-réseau 17b (les diamètres de ces transducteurs peuvent par exemple être respectivement de l'ordre de 8 mm et 1 mm) et adaptés pour délivrer des puissances acoustiques supérieures.

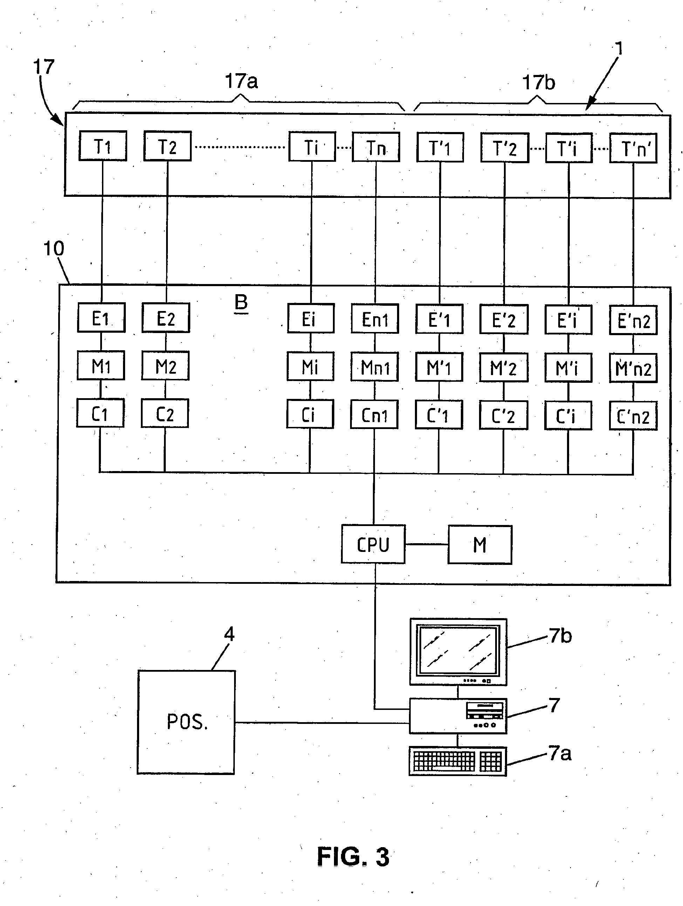

Comme représenté sur la figure 3, la baie électronique 10 qui commande les transducteurs T1-Tn1, T'1-T'n2, peut comprendre :

- une unité centrale électronique CPU commandée par le micro-ordinateur 7 qui commande également le bras robotisé 4 (POS),
- au moins une mémoire centrale M reliée à l'unité centrale électronique CPU,
- des échantillonneurs E1, E2, ... En1, E'1, E'2, ... E'n2 reliés respectivement aux transducteurs T1-Tn1, T'1-T'n2,
- des processeurs ou autres unités centrales électroniques C1, C2, C3, ... Cn1, C'1, C'2, C'3,... C'n2 communiquant avec les échantillonneurs E1-En1, E'1-E'n2,
- et des mémoires M1, M2, M3, ... Mn1, M' 1, M'2, M'3, ... M'n2 reliés respectivement aux processeurs C1-Cn1, C'1-C'n2.

Pour mettre en oeuvre le dispositif décrit précédemment, on commence par fixer le cadre de stéréotaxie 8 à l'extérieur du crâne 14 du patient.

Une fois le cadre de stéréotaxie 8 rigidement fixé au crâne 14 du patient, on prend une image tridimensionnelle du crâne 14 du patient, par exemple au moyen d'un scanner tomodensitométrique (non représenté) ou d'un scanner d'un autre type, qui permet d'obtenir une cartographie tridimensionnelle de la porosité $\Phi$ en chaque point du crâne 14 (ou d'un paramètre représentatif de la porosité). Cette image est avantageusement réalisée avec une résolution fine, par exemple d'environ 0,2 mm.

Pour obtenir cette cartographie de porosité, les valeurs brutes obtenues par le scanner tomodensitométrique peuvent être converties en unités Hounsfield H définies par :

$$H = 1000 \frac{\mu_x - \mu_{eau}}{\mu_{os} - \mu_{eau}} \qquad (1)$$

où $\mu_x$, $\mu_{os}$ et $\mu_{eau}$ désignent respectivement les coefficients d'atténuation linéaire photo-électrique du tissu exploré, de l'os et de l'eau vis à vis des rayons X.

Le crâne pouvant être considéré, du point de vue de l'absorption des rayons X, comme un tissu constitué d'os dont les porosités sont remplies d'eau, on peut considérer que la porosité $\Phi$ du crâne est reliée au coefficient $\mu_x$ par la formule :

$$\mu_x = \Phi \mu_{eau} + (1 - \Phi) \mu_{os} \qquad (2)$$

La porosité en chaque point du crâne 14 est donc directement reliée à la valeur H par la formule :

$$\Phi = 1 - \frac{H}{1000} . \qquad (3)$$

On peut donc connaître la porosité du crâne 14 ou un paramètre représentatif de cette porosité (par exemple la masse volumique ou la densité, parfois donnée directement par l'image scanner avec certains types de scanners) à partir de l'image donnée par le scanner.

A partir des valeurs de porosité $\Phi$ en chaque point du crâne 14, on peut déterminer des cartographies tridimensionnelles de densité massique p, de célérité c des ondes acoustiques et d'absorption $\tau$ desdites ondes acoustiques, en tous points du crâne 14.

La masse volumique p peut être calculée par la formule :

$$\rho = \Phi \times \rho_{eau} + (1 - \Phi) \times \rho_{os} \qquad (4)$$

où $\rho_{eau}$ est la masse volumique de l'eau (1000 Kg.m$^{-3}$) et $\rho_{os}$ est la masse volumique maximale de l'os cortical, qui peut être estime à environ 2100 Kg.m$^{-3}$.

la célérité des ondes acoustiques aux fréquences considérées peut être estimée par la formule :

$$c = c_{min} + (c_{max} - c_{min}) \times (1 - \Phi) \qquad (5)$$

où $C_{min}$ correspond à la célérité des ondes acoustiques dans l'eau (environ 1,5 mm. $\mu s^{-1}$) et $C_{max}$ est la célérité des ondes acoustiques dans l'os cortical (de l'ordre de 2,9 mm.$\mu s^{-1}$).

L'absorption en chaque point du crâne 14 peut être donnée par la formule :

$$\tau = \tau_{min} + (\tau_{max} - \tau_{min}) \times (\Phi)^{\beta} . \qquad (6)$$

Où $\tau_{min}$ correspond à l'absorption minimum des ondes acoustiques dans l'os cortical (par exemple, de l'ordre de 0,1 à 0,5 dB.mm$^{-1}$, notamment de l'ordre de 0, 2 dB.mm$^{-1}$), $\tau_{max}$ correspond à l'absorption des ondes acoustiques dans l'os cortical (par exemple, de l'ordre de 5 à 15 dB.mm$^{-1}$, notamment de l'ordre de 8 dB.mm$^{-1}$) et $\beta$ est un coefficient constant compris par exemple entre 0,3 et 0,7, par exemple égal à 0,5.

Ces différentes cartographies tridimensionnelles de la masse volumique, de la célérité et de l'absorption peuvent être par exemple calculées dans le micro-ordinateur 7 susmentionné, ou dans toute autre unité de calcul, à partir de l'image tridimensionnelle obtenue par scanner tomodensitométrique ou autre. On notera que l'image obtenue par scanner tomodensitométrique ou autre donne également la position relative précise du cadre de stéréotaxie 8 par rapport au crâne 14.

A partir des différentes cartographies susmentionnées, le micro-ordinateur 7 ou tout autre unité de calcul, peut simuler la propagation des ondes acoustiques dans le crâne 14 et le cerveau 19, en considérant le cerveau 19 comme un milieu homogène, assimilable en première approximation à de l'eau pour son comportement vis-à-vis des ondes acoustiques ultrasonores.

Pour effectuer cette simulation, un utilisateur indique au micro-ordinateur quelle doit être le positionnement prévu du réseau de transducteurs 17 à la surface du crâne 14, de façon que le micro-ordinateur puisse simuler la propagation d'ondes acoustiques ultrasonores entre différents points 18 du cerveau 19, dits points de référence, et les emplacements destinés à être occupés par les différents transducteurs T1-Tn1, T'1-T'n2 du réseau 17.

Cette' simulation peut être effectuée en utilisant une équation d'ondes telle que l'équation (7) ci-après :

$$\left(1+\tau\,(\vec{r})\frac{\partial}{\partial t}\cdot\right)\left[\rho\,(\vec{r})\nabla\cdot\left(\frac{1}{\rho\,(\vec{r})}\nabla p(\vec{r},t)\right)\right]-\frac{1}{c\,(\vec{r})^2}\frac{\partial^2 p(\vec{r},t)}{\partial t^2}=S\,(\vec{r},t) \quad （7）\,,$$

où $\overline{r}$ désigne un vecteur de position du point considéré, p désigne la pression et S désigne les signaux acoustiques générés par une source acoustique éventuellement présente au point considéré.

La propagation ,des ondes ultrasonores dans le crâne 14 et le cerveau 19 peut être simulée dans le micro-ordinateur 7 par différences finies, en discrétisant l'équation (7) ci-dessus. Cette simulation pourrait également être effectuée par éléments finis, ou par une méthode de diffraction impulsionnelle.

Avantageusement, ce calcul de propagation d'ondes peut être allégé en effectuant le calcul par simple tracé de rayons dans le cerveau 19 et par différences finies dans le crâne 14 et au voisinage du crâne, en simulant l'émission d'une onde acoustique sphérique à l'interface entre les zones correspondant à ces deux modes de calcul (l'onde sphérique en question est émise vers le cerveau pour simuler une propagation d'ondes provenant des transducteurs, et vers le crâne pour simuler une propagation d'ondes venant du cerveau).

Grâce à cette simulation, il est possible de déterminer de façon non invasive, c'est-à-dire sans devoir nécessairement pénétrer dans le cerveau 19 du patient (sans exclure naturellement une telle pénétration, par exemple lorsqu'il est nécessaire de faire une biopsie), des signaux acoustiques, élémentaires à émettre par les différents transducteurs T1-Tn du réseau 17 afin d'obtenir un champ objectif prédéterminé d'ondes acoustiques dans le cerveau 19.

Ce champ objectif peut par exemple être focalisé en un ou plusieurs points de référence 18 du cerveau 19 et/ou il peut s'agir d'un champ d'ondes plus complexe. Dans tous les cas, la détermination des signaux élémentaires à émettre pour focaliser précisément les ondes acoustiques en différents points de référence 18 du cerveau permet ensuite de générer un champ d'ondes acoustiques plus complexe par combinaison de ces différents signaux acoustiques élémentaires.

Cette détermination peut être réalisée par diverses méthodes, qui font toujours intervenir :

- une étape au cours de laquelle on simule au moins une propagation d'ondes acoustiques entre un ou plusieurs points de référence 18 du cerveau et au moins certains des transducteurs T1-Tn1, T'1-T'n2 du réseau 17 (du cerveau 19 vers le réseau 17 de transducteurs et/ou du réseau 17 vers le cerveau 19) : dans l'exemple considéré, ces simulations sont de préférence effectuées d'une part, entre les points de référence 18 et les transducteurs du sous-réseau de traitement 17a, et d'autre part, entre les points de référence 18 et les transducteurs du sous-réseau d'imagerie 17b ;
- et une étape dans laquelle on calcule à partir de la simulation précédente des signaux acoustiques à émettre par les transducteurs considérés du réseau 17 pour obtenir le champ objectif d'ondes acoustiques dans le cerveau 19.

Dans tous les cas de figure, la détermination des signaux acoustiques élémentaires à émettre par les transducteurs T1-Tn pour obtenir un champ objectif d'ondes acoustiques en un ou plusieurs points 18 du cerveau 19 peut être obtenue en une période comprise, entre quelques dizaines de minutes et quelques heures suivant la puissance de calcul disponible et suivant le nombre de points de référence 18 considérés. Les signaux acoustiques ainsi déterminés peuvent permettre d'obtenir notamment des focalisations d'ondes acoustiques très précises, sur des taches focales par exemple de l'ordre de 1 mm de diamètre, ce qui permet une excellente précision en imagerie échographique comme en traitement par hyperthermie (à titre d'exemple, la précision des traitements de tumeurs, par radiothérapie est de l'ordre de quelques centimètres, donc beaucoup moins précise, et entraîne en outre des effets secondaires contrairement aux traitement par hyperthermie).

On notera par ailleurs qu'après l'étape de détermination des signaux acoustiques élémentaires' à émettre par les transducteurs T1-Tn1, T'1-T'n2, le procédé et le dispositif selon l'invention peuvent permettre de tester virtuellement l'émission, par le réseau 17 de transducteurs, de signaux acoustiques correspondant à un ou plusieurs champs objectifs d'ondes acoustiques dans le cerveau 19, de façon à vérifier par le calcul que la propagation d'ondes acoustiques dans le crâne. 14 et le cerveau 19 satisfaits certains critères prédéfinis, notamment la qualité de la focalisation, l'obtention de certaines plages de température par l'échauffement dû aux ondes acoustiques, l'absence de cavitation susceptible de générer des lésions, etc.

Pour simuler l'évolution temporelle et spatiale de la température du fait de l'apport de chaleur par le faisceau ultrasonore, on peut par exemple utiliser l'équation de diffusion suivante :

$$\rho c_p \frac{\partial T(\vec{r},t)}{\partial t} - K \, \Delta T(\vec{r},t) = \mu \frac{p^2(\vec{r},t)}{\rho_0(\vec{r})c_0(\vec{r})}$$

où $cp$ représente la capacité calorifique du milieu, $K$ sa conductivité thermique et $\mu$ son coefficient d'absorption. Le terme source de l'équation de diffusion correspond à l'apport due chaleur dans le milieu par le champ de pression dû aux ondes acoustiques.

La distribution spatiale de pression $p(r, t)$ dans le milieu (crâne + cerveau) ayant été modélisée grâce à la simulation numérique de propagation d'ondes, ces données peuvent être injectées dans une simulation numérique par différences finies de l'équation de diffusion de la chaleur citée ci-dessus. On peut donc ainsi prédire avant le traitement la distribution de température au cours du traitement par hyperthermie par le réseau de traitement 17a et vérifier par exemple que l'élévation de température n'est pas trop importante dans l'os ou tout autre zone sensible que l'on cherche à préserver.

La détermination des signaux acoustiques élémentaires susmentionnés pour obtenir le champ objectif d'ondes acoustiques peut être effectuée par diverses méthodes.

Selon une première méthode :

- on simule une propagation d'ondes acoustiques depuis au moins un point de référence 18 du cerveau vers chaque transducteur T1-Tn1 ou T'1-T'n2 du sous-réseau de transducteurs considéré et on détermine des signaux acoustiques simulés reçus Ri(t) arrivant à l'emplacement de chaque transducteur i du réseau de transducteurs appartenant au sous-réseau considéré, i étant un entier compris entre 1 et n et n étant le nombre de transducteurs du réseau 17 de transducteurs,
- et on détermine les signaux acoustiques élémentaires à émettre Ei(t) comme étant proportionnels à une inversion temporelle Ri(-t) desdits signaux acoustiques simulés reçus Ri(t), précédemment déterminés.

Les signaux acoustiques à émettre Ei(t) sont ainsi calculé par la formule : Ei(t)=Gi . Ri(-t), où Gi est un facteur de gain qui peut être :

- identique pour tous les transducteurs (éventuellement égal à 1),
- ou différent d'un transducteur i à l'autre, pour compenser les dissipations dans le crâne.

Lorsque les facteurs de gain Gi sont différents d'un transducteur à l'autre, ils peuvent être calculés par la formule : Gi=a/Max(|Ri(t)|)², où a est un nombre réel commun à tous les transducteurs du réseau de transducteurs et Max(|Ri(t)|) est une valeur maximale de l'amplitude du signal Ri(t). Avantageusement, Le gain Gi ne prend cette valeur que pour les transducteurs i recevant un signal d'amplitude suffisante (par exemple lorsque Max(|Ri(t)|) est au moins égal à 10% de la plus grande valeur Max(|Ri(t)|) pour tous les transducteurs i considérés) et est égal à 1 sinon.

Selon une deuxième méthode, on procède comme précédemment avec Gi=1, mais on effectue la simulation de propagation d'ondes acoustiques en utilisant une cartographie tridimensionnelle fictive d'absorption des ondes acoustiques, ayant en chaque point de la barrière osseuse des coefficients d'absorption -τ opposés aux coefficients d'absorption réels τ déterminés au cours de l'étape (1b) : les dissipations dans le milieu sont ainsi automatiquement compensées.

Selon une troisième méthode, on procède initialement comme dans la première méthode susmentionnée pour calculer les signaux Ri(t) susmentionnés,

- puis on simule une émission par chaque transducteur i considéré d'un signal acoustique Ri(-t) correspondant à une inversion temporelle du signal Ri(t), et une propagation dans le produit fluide du réservoir 13 jusqu'à un transducteur fictif Fi (voir figure 2) situé au contact du crâne en correspondance avec le transducteur i, et on détermine des signaux acoustiques simulés reçus R'i(t) arrivant à l'emplacement dudit transducteur fictif i,

- puis on simule une émission par chaque transducteur fictif i d'un signal acoustique G'i.R'i(-t) où R'i(-t) est une inversion temporelle du signal R'i(t) et G'i est un coefficient inversement proportionnel au carré d'une amplitude du signal R'i(t) au moins pour certains transducteurs fictifs i (par exemple : Gi=a/Max(|Ri(t)|)$^2$, où a est un nombre réel commun à tous les transducteurs du réseau de transducteurs et Max(|Ri(t)|) est une valeur maximale de l'amplitude du signal Ri(t) ; avantageusement, Le gain Gi ne prend cette valeur que pour les transducteurs i recevant un signal d'amplitude suffisante [par exemple lorsque Max(|Ri(t)|) est au moins égal à 10%, de la plus grande valeur Max(|Ri(t)|) pour tous les transducteurs i considérés] et est égal à 1 sinon),
- puis on simule une propagation dans ledit produit fluide jusqu'au transducteur i, et on détermine des signaux acoustiques simulés reçus R"i(t) arrivant à l'emplacement dudit transducteur i,
- et on détermine les signaux acoustiques élémentaires à émettre Ei(t) comme étant égaux à une inversion temporelle R"i(-t) desdits signaux acoustiques simulés reçus R"i(t).

Selon une quatrième méthode :

- on simule l'émission d'une impulsion d'ondes acoustiques par chaque transducteur i du réseau 17 ou d'un sous-réseau 17a, 17b de transducteurs et une propagation d'ondes acoustiques depuis chaque transducteur i considéré vers plusieurs points de référence r situés dans le cerveau, i étant un indice compris entre 1 et n qui désigne un transducteur du réseau et n étant un entier naturel non nul qui désigne le nombre total de transducteurs, r étant un entier compris entre 1 et.'m et m étant un entier naturel non nul désignant le nombre de points de référence 18,
- on détermine des réponses impulsionnelles simulées hri(t) arrivant en chacun desdits points' de référence r du cerveau,
- et l'étape de calcul des signaux acoustiques élémentaires comprend les sous-étapes suivantes :

    (1e1) on détermine un nombre p de composantes fréquentielles de chacune de ces réponses impulsionnelles simulées, de fréquences respectives ωk, k étant un indice compris entre 1 et p qui désigne une composante fréquentielles,
    (1e2) on détermine p matrices de transfert H(ωk)=[Hri(ωk)], i allant de 1 à n et r allant de 1 à m, où Hri(ωk) est la valeur à la fréquence ωk, de la transformée de Fourier de la réponse impulsionnelle Hri(t),
    (1e3) on détermine, pour chaque point de référence r, n composantes Ei(ωk, r) telles que F(ωk,r)=H(ωk).E(ωk,r), où E(ωk,r) = [Ei(ωk, r)] est un vecteur à n composantes Ei(ω, r), F(ωk, r) = [F1 (ωk, r)] est un vecteur à m composantes Fl(ωk, r) où 1, varie entre 1 et m, ces m composantes Fl (ωk, r) correspondant à la génération dudit champ objectif prédéterminé d'ondes acoustiques à la fréquence ωk aux points r.

Au cours de la sous-étape (le3), on peut par exemple calculer p matrice H$^{-1}$(ωk) au moins par inversion des matrices de transfert de H(ωk), et pour chaque point de référence r (18) du cerveau, on calcule le vecteur E(ωk, r) par la formule : E (ωk, r)= H$^{-1}$(ωk). F (ωk, r).

Au cours de la phase d'apprentissage qui vient d'être décrite, quelle que soit la méthode utilisée, on détermine également de préférence des réponses impulsionnelles hir(t) entre plusieurs point de référence r (18) du cerveau et chaque transducteur i du réseau d'imagerie 17b (c'est à dire pour des ondes acoustiques se propageant du cerveau vers le réseau 17 de transducteurs), et on détermine également comment focaliser le réseau d'imagerie 17b en réception sur chaque point de référence 18 pour réaliser une image échographique.

On peut ainsi utiliser le dispositif 1 à des fins d'imagerie échographique, en déconvoluant des signaux acoustiques rétro-diffusés par le cerveau 19. Cette déconvolution peut être effectuée par tout moyen connu, par exemple en travaillant dans le domaine fréquentiel après transformée de Fourier, sur un nombre p de composantes fréquentielles monochromatiques ωk, par inversion de la matrice de transfert H(ωk)=[Hir (ωk)], dont les composantes Hir(ωk) sont les composantes fréquentielles des transformées de Fourier des réponses impulsionnelles hir(t) à la fréquence ωk. On peut ainsi obtenir les signaux rétro-diffusés par chaque point du cerveau, et notamment l'amplitude de ces signaux (par exemple, la valeur maximale de l'amplitude), ce qui permet de réaliser une image échographique précise du cerveau 19.

Une fois que les signaux acoustiques élémentaires susmentionnés ont été déterminé par simulation, on positionne le réseau réel 17 de transducteurs acoustiques à l'extérieur du crâne 14 du patient, exactement dans la même position que celle utilisée lors de la simulation numérique.

Afin de garantir que le réseau 17 de transducteurs se trouve dans la même position que la position utilisée lors de la simulation numérique, on positionne d'abord le casque 2 portant le réseau de transducteurs dans une position approximativement correcte par rapport au crâne 14, puis on fait repérer la position réelle du casque 2 par rapport au crâne par le sous-réseau d'imagerie 17b par imagerie échographique.

Ce repérage peut être effectué :

- en repérant par échographie une position relative entre le réseau de transducteurs et les parties du cadre de stéréotaxie 8 qui sont au contact de la paroi souple 12 du casque,

- et/ou en repérant directement la position relative entre ledit réseau de transducteurs et le crâne 14, en déterminant une forme extérieure d'au moins une partie du crâne 14 par échographie, puis en comparant cette forme extérieure à l'image tridimensionnelle du crâne (cette dernière méthode peut être utilisée seule notamment si l'on peut par ailleurs se passer du cadre de stéréotaxie 8).

A partir de cette position relative, le micro-ordinateur 7 peut déplacer le casque 2 au moyen du bras robotisé, de façon que sa position corresponde exactement à celle utilisée pour les simulations.

Une fois effectué le positionnement du réseau 17 de transducteurs par rapport au crâne 14, le praticien peut à volonté faire générer des champs objectifs d'ondes acoustiques dans le cerveau 19 du patient, en fonction de ses desiderata, par exemple à des fins d'imagerie et/ou de traitement par hyperthermie.

Lorsque le praticien souhaite réaliser un traitement par hyperthermie d'une ou plusieurs zones du cerveau, il peut focaliser très précisément les ondes acoustiques sur la ou les zones à traiter (dans le cas où il y aurait plusieurs zones à traiter, cette focalisation peut être faite successivement ou parallèlement).

Dans tous les cas, le traitement par hyperthermie se fait avantageusement par périodes successives discontinues, de façon à éviter un échauffement trop prolongé qui conduirait à une diffusion thermique susceptible de créer des lésions en dehors des zones à détruire. Entre les périodes de traitement par hyperthermie, on peut réaliser, comme expliqué précédemment, des images échographiques successives du cerveau.

Ces images ' échographiques peuvent être avantageusement traitées par le micro-ordinateur 7 de façon à obtenir une carte de température du cerveau, notamment par le procédé connu sous le nom de « compound imaging », ce qui permet de visualiser l'efficacité du traitement par hyperthermie. Ce procédé a été décrit notamment par M. Pernot et al ("Improvement of Ultrasound Based Temperature Estimation by Compound Imaging", Proceedings of the International Symposium on Therapeutic Ultrasound, Seattle 2002) et Entrekin etal. (Medica Mundi, vol. 43, Sept. 1999, p. 35-43, "Real Time Spatial Compound Imaging in breast ultrasound: technology and early clinical experience").

**Revendications**

1. Procédé non invasif pour obtenir au moins un champ objectif prédéterminé d'ondes acoustiques dans un milieu sensiblement homogène (19) masqué par une barrière osseuse (14), en faisant émettre des signaux acoustiques par au moins un réseau (17) de transducteurs à travers ladite barrière osseuse (14), **caractérisé en ce qu'**il comporte :

   - une phase d'apprentissage comprenant au moins les étapes suivantes :

      (1a) réaliser, au moins partiellement par rayons X, une image tridimensionnelle de la barrière osseuse (14), donnant un paramètre représentatif de la porosité de ladite barrière osseuse en différents points,
      (1b) à partir de ladite image tridimensionnelle, déterminer au moins des cartographies tridimensionnelles de masse volumique, de célérité des ondes acoustiques et d'absorption des ondes acoustiques dans ladite barrière osseuse,
      (1c) déterminer un positionnement prévu du réseau (17) de transducteurs par rapport à la barrière osseuse (14),
      (1d) simuler au moins une propagation d'ondes acoustiques entre au moins un point du milieu sensiblement homogène et au moins certains transducteurs du réseau de transducteurs, à partir d'un modèle mathématique de propagation et desdites cartographies tridimensionnelles de masse volumique, de célérité des ondes acoustiques et d'absorption des ondes acoustiques,
      (1e) à partir de ladite simulation, calculer des signaux acoustiques élémentaires à émettre par au moins certains transducteurs dudit réseau de transducteurs pour obtenir ledit champ objectif d'ondes acoustiques,

   - et une phase de positionnement réel du réseau de transducteurs sur la barrière osseuse, comprenant les étapes suivantes :

      (2a) positionner d'abord approximativement le réseau (17) de transducteurs sur la barrière osseuse (14), dans sa position prévue,
      (2b) faire repérer par au moins certains transducteurs du réseau de transducteurs, par échographie, une position relative entre ledit réseau (17) de transducteurs et la barrière osseuse (14),
      (2c) et affiner la position du réseau (17) de transducteurs par rapport à ladite barrière osseuse en fonction du repérage effectué à l'étape (2b).

**2.** Procédé selon la revendication 1, dans lequel, au cours de l'étape (2b), on repère la position relative entre ledit réseau (17) de transducteurs et la barrière osseuse (14), en déterminant une forme extérieure d'au moins une partie de la barrière osseuse par échographie, par au moins une partie dudit réseau de transducteurs, en comparant cette forme extérieure à ladite image tridimensionnelle de la barrière osseuse.

**3.** Procédé selon la revendication 1, dans lequel :

- l'étape (1a) est précédée d'une étape initiale au cours de laquelle on fixe rigidement un dispositif de repérage (8) sur ladite barrière osseuse (14), ledit dispositif de repérage (8) étant adapté pour absorber des rayons X,
- au cours de l'étape (1a), l'image tridimensionnelle réalisée donne également un positionnement du dispositif de repérage (8) sur la barrière osseuse (14),
- et au cours de l'étape (2b), on repère par échographie une position relative entre le réseau (17) de transducteurs et le dispositif de repérage (8).

**4.** Procédé selon l'une quelconque des revendications précédentes, dans lequel le réseau de transducteurs est inclus dans un réservoir (13) rempli de produit fluide et comprenant au moins une paroi souple (12), et au cours de la phase de positionnement, cette paroi souple est placée en appui contre la barrière osseuse (14).

**5.** Procédé selon l'une quelconque des revendications précédentes, dans lequel le milieu sensiblement homogène (19) comprend au moins une partie d'un cerveau masqué par une barrière osseuse (14) comprenant au moins une partie d'un crâne entourant ce cerveau.

**6.** Procédé selon l'une quelconque des revendications précédentes, dans lequel les ondes acoustiques ont des fréquences comprises entre 0,5 et 3 MHz.

**7.** Procédé selon l'une quelconque des revendications précédentes, dans lequel l'étape (1e) est suivie d'une étape (1f) au cours de laquelle on simule au moins une émission, par le réseau (17) de transducteurs, de signaux acoustiques déterminés à partir desdits signaux acoustiques élémentaires et permettant d'obtenir un champ d'ondes acoustiques souhaité, on simule une propagation d'ondes acoustiques générées par cette émission, et on vérifie que cette propagation satisfait à certains critères prédéfinis.

**8.** Procédé selon l'une quelconque des revendications précédentes, dans lequel :

- au cours de l'étape (1d), on simule une propagation d'ondes acoustiques depuis au moins un point (18) du milieu sensiblement homogène vers au moins certains transducteurs du réseau de transducteurs et on détermine des signaux acoustiques simulés reçus $R_i(t)$ arrivant à l'emplacement desdits transducteurs i du réseau de transducteurs, i étant un entier compris entre 1 et n et n étant le nombre de transducteurs du réseau de transducteurs,
- et au cours de l'étape (1e), on détermine les signaux acoustiques élémentaires $E_i(t)$, à émettre par chaque transducteur i considéré, comme étant proportionnels à une inversion temporelle $R_i(-t)$ desdits signaux acoustiques simulés reçus $R_i(t)$, précédemment déterminés à l'étape (1d).

**9.** Procédé selon la revendication 8, dans lequel, au cours de l'étape (1e), on détermine les signaux acoustiques à émettre $E_i(t)$ par la formule : $E_i(t) = G_i.R_i(-t)$, où $G_i$ est un facteur de gain différent d'un transducteur i à l'autre, pour compenser les dissipations dans la barrière osseuse.

**10.** Procédé selon la revendication 9, dans lequel les facteurs de gain $G_i$ correspondant à au moins certains transducteurs sont respectivement inversement proportionnels au carré d'une amplitude des signaux acoustiques simulés reçus $R_i(t)$ correspondants.

**11.** Procédé selon la revendication 8, dans lequel :

- au cours de l'étape (1d), on effectue la simulation en utilisant une cartographie tridimensionnelle fictive d'absorption des ondes acoustiques, ayant en chaque point de la barrière osseuse des coefficients d'absorption -$\tau$ opposés à des coefficients d'absorption réels $\tau$ déterminés au cours de l'étape (1b),
- et au cours de l'étape (1e), on détermine les signaux acoustiques élémentaires à émettre $E_i(t)$ comme étant égaux à ladite inversion temporelle $R_i(-t)$.

**12.** Procédé selon l'une quelconque des revendications 1 à 7, dans lequel le réseau (17) de transducteurs est inclus dans un réservoir (13) rempli de produit fluide et comprenant au moins une paroi souple (12) destinée à être placée en appui contre la barrière osseuse (14), les emplacements prévus des transducteurs, déterminés au cours de l'étape (1c), n'étant pas au contact de la barrière osseuse, et dans lequel :

au cours de l'étape (1d) :

- on simule une propagation d'ondes acoustiques depuis au moins un point (18) du milieu sensiblement homogène (19) vers au moins certains transducteurs du réseau (17) de transducteurs, et on détermine des signaux acoustiques simulés reçus Ri(t) arrivant à l'emplacement desdits transducteurs i du réseau de transducteurs, i étant un entier compris entre 1 et n et n étant le nombre de transducteurs du réseau de transducteurs,
- puis on simule une émission par chaque transducteur i d'un signal acoustique Ri(-t) correspondant à une inversion temporelle du signal Ri(t), et une propagation dans ledit produit fluide jusqu'à un transducteur fictif i situé au contact de la barrière osseuse (14) en correspondance avec le transducteur i, et on détermine des signaux acoustiques simulés reçus R'i(t) arrivant à l'emplacement dudit transducteur fictif i,
- puis on simule une émission par chaque transducteur fictif i d'un signal acoustique G'i.R'i(-t) où R'i(-t) est une inversion temporelle du signal R'i(t) et G'i est un coefficient inversement proportionnel au carré d'une amplitude du signal R'i(t) au moins pour certains transducteurs fictifs i,
- puis on simule une propagation dans ledit produit fluide jusqu'au transducteur i, et on détermine des signaux acoustiques simulés reçus R''i(t) arrivant à l'emplacement dudit transducteur i,

et au cours de l'étape (1e), on détermine les signaux acoustiques élémentaires à émettre Ei (t) comme étant égaux à une inversion temporelle R''i(-t) desdits signaux acoustiques simulés reçus R''i(t).

**13.** Procédé selon l'une quelconque des revendications 1 à 7, dans lequel au cours de l'étape (1d), on simule l'émission d'une impulsion d'ondes acoustiques par au moins certains transducteurs i du réseau (17) de transducteurs et une propagation d'ondes acoustiques depuis chaque transducteur i considéré vers plusieurs points de référence r situés dans le milieu sensiblement homogène (19), i étant un indice compris entre 1 et n qui désigne un transducteur du réseau et n étant un entier naturel non nul qui désigne le nombre de transducteurs, r étant un entier compris entre 1 et m et m étant un entier naturel non nul désignant le nombre de points de référence, et on détermine des réponses impulsionnelles simulées hri(t) arrivant en chacun desdits points de référence r du milieu sensiblement homogène, l'étape (1e) comprenant les sous-étapes suivantes :

(1e1) on détermine un nombre p de composantes fréquentielles de chacune de ces réponses impulsionnelles simulées, de fréquences respectives $\omega k$, k étant un indice compris entre 1 et p qui désigne une composante fréquentielle,
(1e2) on détermine p matrices de transfert $H(\omega k)=[Hri(\omega k)]$, i allant de 1 à n et r allant de 1 à m, où $Hri(\omega k)$ est la valeur à la fréquence $\omega k$, de la transformée de Fourier de la réponse impulsionnelle Hri(t),
(1e3) on détermine, pour chaque point de référence r, n composantes $Ei(\omega k,r)$ telles que $F(\omega k,r)=H(\omega k).E(\omega k,r)$, où $E(\omega k,r)=[Ei(\omega k,r)]$ est un vecteur à n composantes $Ei(\omega k,r)$, $F(\omega k,r)=[Fl(\omega k,r)]$ est un vecteur à m composantes $Fl(\omega k,r)$ où l varie entre 1 et m, ces m composantes $Fl(\omega k,r)$ correspondant à la génération dudit champ objectif prédéterminé d'ondes acoustiques à la fréquence $\omega k$ aux points r.

**14.** Procédé selon la revendication 13, dans lequel, au cours de la sous-étape (1e3), on calcule p matrices $H^{-1}(\omega k)$ au moins par inversion des matrices de transfert de $H(\omega k)$, et pour chaque point de référence r du milieu sensiblement homogène, on calcule le vecteur $E(\omega k, r)$ par la formule :

$$E(\omega k, r) = H^{-1}(\omega k) . F(\omega k, r).$$

**15.** Procédé selon l'une quelconque des revendications précédentes, dans lequel, au cours de l'étape' (1d), on détermine des réponses impulsionnelles hir(t) entre plusieurs points de référence r du milieu sensiblement homogène et au moins certains transducteurs i du réseau (17) de transducteurs, i étant un indice compris entre 1 et n qui désigne un transducteur et n étant un entier naturel non nul qui désigne le nombre de transducteurs, r étant un entier compris entre 1 et m et m étant un entier naturel non nul désignant le nombre de points de référence, et au cours de l'étape (1e), on détermine également comment focaliser au moins une partie du réseau (17) de transducteur en réception sur chaque point de référence r pour réaliser une image échographique.

16. Procédé d'imagerie médicale par échographie, comprenant un procédé non invasif pour obtenir un champ objectif d'ondes acoustiques selon l'une quelconque des revendications précédentes, et une phase d'imagerie au cours de laquelle on réalise au moins une image échographique du milieu sensiblement homogène (19) à l'aide d'au moins une partie du réseau (17) de transducteurs, en utilisant les signaux acoustiques élémentaires déterminés au cours de la phase d'apprentissage.

17. Dispositif spécialement conçu pour la mise en oeuvre d'un procédé selon l'une quelconque des revendications précédentes, ce dispositif comprenant au moins :

- un réseau (17) de transducteurs adapté pour être positionné à l'extérieur d'une barrière osseuse (14) masquant un milieu sensiblement homogène (19),
- des moyens (7) pour déterminer au moins des cartographies tridimensionnelles de masse volumique, de célérité des ondes acoustiques et d'absorption des ondes acoustiques dans la barrière osseuse, à partir d'une image tridimensionnelle de ladite barrière osseuse réalisée par rayons X et donnant la porosité de ladite la barrière osseuse en chaque point,
- des moyens (7) pour simuler au moins une propagation d'ondes acoustiques entre au moins un point (18) du milieu sensiblement homogène et au moins certains transducteurs du réseau (17) de transducteurs, à partir d'un modèle mathématique de propagation et desdites cartographies tridimensionnelles de masse volumique, de célérité des ondes acoustiques et d'absorption des ondes acoustiques, et en fonction d'un positionnement prévu du réseau (17) de transducteurs par rapport à la barrière osseuse (14),
- des moyens (7) pour calculer, à partir de ladite simulation, des signaux acoustiques élémentaires à émettre par au moins certains transducteurs dudit réseau (17) de transducteurs pour obtenir un champ objectif d'ondes acoustiques dans le milieu sensiblement homogène,
- des moyens (7) pour faire repérer par au moins certains transducteurs du réseau (17) de transducteurs, par échographie, une position relative entre ledit réseau (17) de transducteurs et la barrière osseuse (14),
- et des moyens (7, 4) pour affiner une position relative initiale du réseau (17) de transducteurs par rapport à la barrière osseuse (14) en fonction du repérage de la position relative entre le réseau de transducteurs et la barrière osseuse, de façon que ladite position relative du réseau de transducteur par rapport à la barrière osseuse corresponde au positionnement prévu.

18. Dispositif selon la revendication 17, dans lequel les moyens pour repérer la position relative entre ledit réseau (17) de transducteurs et la barrière osseuse (14) sont adaptés pour déterminer une forme extérieure d'au moins une partie de la barrière osseuse (14) par échographie, par l'intermédiaire d'au moins une partie dudit réseau (17) de transducteurs, en comparant cette forme extérieure à ladite image tridimensionnelle de la barrière osseuse.

19. Dispositif selon la revendication 17 ou la revendication 18, comprenant un dispositif de repérage (8) doté de moyens de fixation (16) adaptés pour fixer rigidement ledit dispositif de repérage sur la barrière osseuse (14), et ledit dispositif de repérage étant adapté pour absorber des rayons X et être visible sur l'image tridimensionnelle de ladite barrière osseuse, et les moyens (7) pour repérer la position relative entre ledit réseau (17) de transducteurs et la barrière osseuse (14) sont adaptés pour repérer par échographie une position relative entre le réseau (17) de transducteurs et le dispositif de repérage (8).

20. Dispositif selon l'une quelconque des revendications 17 à 19, dans lequel le réseau (17) de transducteurs est inclus dans un réservoir (13) rempli de produit fluide et comprenant au moins une paroi souple (12) destinée à venir en appui contre la barrière osseuse (14).

21. Dispositif selon l'une quelconque des revendications 17 à 20, dans lequel le réseau (17) de transducteurs comprend un sous-réseau de traitement par hyperthermie (17a) et un sous-réseau d'imagerie (17b), ces deux sous-réseaux comportant respectivement des transducteurs de types différents.

22. Dispositif selon l'une quelconque des revendications 17 à 21, dans lequel le réseau (17) de transducteurs est adapté pour émettre des ondes acoustiques ayant des fréquences comprises entre 0,5 et 3 MHz.

23. Dispositif selon l'une quelconque des revendications 17 à 22, comprenant en outre des moyens de simulation (7) adaptés pour :

- simuler au moins une émission, par le réseau (17) de transducteurs, de signaux acoustiques déterminés à partir desdits signaux acoustiques élémentaires et permettant d'obtenir un champ d'ondes acoustiques souhaité,
- simuler une propagation d'ondes acoustiques générées par cette émission,
- et vérifier que cette propagation satisfait à certains critères prédéfinis.

**24.** Dispositif selon l'une quelconque des revendications 17 à 23, dans lequel :

les moyens pour simuler (7) sont adaptés pour simuler une propagation d'ondes acoustiques depuis au moins un point (18) du milieu sensiblement homogène vers au moins certains transducteurs du réseau de transducteurs déterminer des signaux acoustiques simulés reçus Ri(t) arrivant à l'emplacement desdits transducteurs i du réseau de transducteurs, i étant un entier compris entre 1 et n et n étant le nombre de transducteurs du réseau de transducteurs,

et les moyens pour calculer (7) sont adaptés pour déterminer les signaux acoustiques élémentaires Ei(t), à émettre par chaque transducteur i considéré, comme étant proportionnels à une inversion temporelle Ri(-t) desdits signaux acoustiques simulés reçus Ri(t), précédemment déterminés à l'étape (1d).

**25.** Dispositif selon la revendication 24, dans lequel les moyens pour calculer (7) sont adaptés pour déterminer les signaux acoustiques à émettre Ei(t) par la formule : Ei(t) = Gi.Ri(-t), où Gi est un facteur de gain différent d'un transducteur i à l'autre, pour compenser les dissipations dans la barrière osseuse.

**26.** Dispositif selon la revendication 25, dans lequel les facteurs de gain Gi correspondant à au moins certains transducteurs sont respectivement inversement proportionnels au carré d'une amplitude des signaux acoustiques simulés reçus Ri(t) correspondants.

**27.** Dispositif selon la revendication 24, dans lequel :

les moyens pour simuler (7) sont adaptés pour effectuer ladite simulation de propagation d'onde acoustique en utilisant une cartographie tridimensionnelle fictive d'absorption des ondes acoustiques, ayant en chaque point de la barrière osseuse des coefficients d'absorption -$\tau$ opposés à des coefficients d'absorption réels $\tau$ déterminés au cours de l'étape (1b),

et les moyens pour calculer (7) sont adaptés pour déterminer les signaux acoustiques élémentaires à émettre Ei(t) comme étant égaux à ladite inversion temporelle Ri(-t).

**28.** Dispositif selon l'une quelconque des revendications 17 à 23, dans lequel le réseau (17) de transducteurs est inclus dans un réservoir (13) rempli de produit fluide et comprenant au moins une paroi souple (12) destinée à être placée en appui contre la barrière osseuse (14), les emplacements prévus des transducteurs, pris en compte par les moyens pour simuler (7), n'étant pas au contact de la barrière osseuse,

dans lequel les moyens pour simuler (7) sont adaptés pour :

- simuler une propagation d'ondes acoustiques depuis au moins un point (18) du milieu sensiblement homogène (19) vers au moins certains transducteurs du réseau (17) de transducteurs et déterminer des signaux acoustiques simulés reçus Ri(t) arrivant à l'emplacement desdits transducteurs i du réseau de transducteurs, i étant un entier compris entre 1 et n et n étant le nombre de transducteurs du réseau de transducteurs,
- simuler une émission par chaque transducteur i d'un signal acoustique Ri(-t) correspondant à une inversion temporelle du signal Ri(t), et une propagation dans ledit produit fluide jusqu'à un transducteur fictif i situé au contact de la barrière osseuse (14) en correspondance avec le transducteur i, et déterminer des signaux acoustiques simulés reçus R'i(t) arrivant à l'emplacement dudit transducteur fictif i,
- simuler une émission par chaque transducteur fictif i d'un signal acoustique G'i.R'i(-t) où R'i(-t) est une inversion temporelle du signal R'i(t) et G'i est un coefficient inversement proportionnel au carré d'une amplitude du signal R'i(t) au moins pour certains transducteurs fictifs i,
- et simuler une propagation dans ledit produit fluide jusqu'au transducteur i, et déterminer des signaux acoustiques simulés reçus R''i(t) arrivant à l'emplacement dudit transducteur i,

et dans lequel les moyens pour calculer (7) sont adaptés pour déterminer les signaux acoustiques élémentaires à émettre Ei(t) comme étant égaux à une inversion temporelle R''i(-t) desdits signaux acoustiques simulés reçus R''i(t).

**29.** Dispositif selon l'une quelconque des revendications 17 à 23, dans lequel les moyens pour simuler (7) sont adaptés pour :

- simuler l'émission d'une impulsion d'ondes acoustiques par au moins certains transducteurs i du réseau (17) de transducteurs et une propagation d'ondes acoustiques depuis chaque transducteur i considéré vers plusieurs points de référence r situés dans le milieu sensiblement homogène (19), i étant un indice compris entre 1 et n qui désigne un transducteur du réseau et n étant un entier naturel non nul qui désigne le nombre dé transducteurs, r étant un entier compris entre 1 et m et m étant un entier naturel non nul désignant le nombre de points de référence,

- déterminer des réponses impulsionnelles simulées hri(t) arrivant en chacun desdits points de référence r du milieu sensiblement homogène,

et dans lequel les moyens pour calculer (7) sont adaptés pour :

- déterminer un nombre p de composantes fréquentielles de chacune de ces réponses impulsionnelles simulées, de fréquences respectives $\omega k$, k étant un indice compris entre 1 et p qui désigne une composante fréquentielle,

- déterminer p matrices de transfert $H(\omega k)=[Hri(\omega k)]$, i allant de 1 à n et r allant de 1 à m, où $Hri(\omega k)$ est la valeur à la fréquence $\omega k$, de la transformée de Fourier de la réponse impulsionnelle $Hri(t)$,

- et déterminer, pour chaque point de référence r, n composantes $Ei(\omega k,r)$ telles que $F(\omega k,r)=H(\omega k).E(\omega k,r)$, où $E(\omega k,r)=[Ei(\omega k,r)]$ est un vecteur à n composantes $Ei(\omega k,r)$, $F(\omega k,r)=[Fl(\omega k,r)]$ est un vecteur à m composantes $Fl(\omega k,r)$ où 1 varie entre 1 et m, ces m composantes $Fl(\omega k,r)$ correspondant à la génération dudit champ objectif prédéterminé d'ondes acoustiques à la fréquence $\omega k$ aux points r.

**30.** Dispositif selon la revendication 29, dans lequel les moyens pour calculer (7) sont adaptés pour :

- calculer p matrices $H^{-1}(\omega k)$ au moins par inversion des matrices de transfert de $H(\omega k)$,

- et pour chaque point de référence r du milieu sensiblement homogène, calculer le vecteur $E(\omega k, r)$ par la formule :

$$E\ (\omega k, r) = H^{-1}(\omega k).\ F\ (\omega k, r).$$

**31.** Dispositif selon l'une quelconque des revendications 17 à 30, dans lequel les moyens pour simuler (7) sont adaptés pour :

- déterminer des réponses impulsionnelles hir(t) entre plusieurs points de référence r du milieu sensiblement homogène et au moins certains transducteurs i du réseau (17) de transducteurs, i étant un indice compris entre 1 et n qui désigne un transducteur et n étant un entier naturel non nul qui désigne le nombre de transducteurs, r étant un entier compris entre 1 et m et m étant un entier naturel non nul désignant le nombre de points de référence,

et dans lequel les moyens pour calculer (7) sont adaptés pour déterminer comment focaliser au moins une partie du réseau (17) de transducteur en réception sur chaque point de référence r pour réaliser une image échographique.

**32.** Dispositif selon l'une quelconque des revendication 17 à 31, comprenant en outre des moyens d'imagerie (7, 7b) adaptés pour réaliser au moins une image échographique du milieu sensiblement homogène (19) à l'aide d'au moins une partie du réseau (17) de transducteurs, en utilisant les signaux acoustiques élémentaires déterminés par les moyens pour calculer (7).

**Patentansprüche**

**1.** Nicht-invasives Verfahren zum Erhalten wenigstens eines vorbestimmten Soll-Feldes aus akustischen Wellen in einem im Wesentlichen homogenen Medium (19), welches durch eine Knochen-Barriere (14) verdeckt ist, durch Emittieren von akustischen Signalen durch wenigstens eine Anordnung (17) von Transduktoren durch die Knochen-Barriere (14) hindurch,

**dadurch gekennzeichnet, dass** es umfasst:

- eine Lernphase, welche wenigstens die folgenden Schritte umfasst:

(1a) Bilden eines dreidimensionalen Bildes der Knochen-Barriere (14), wenigstens teilweise mittels Röntgen-Strahlen, wodurch ein die Porosität der Knochen-Barriere in verschiedenen Punkten repräsentierender Parameter erhalten wird,

(1b) Bestimmen wenigstens von dreidimensionalen Karten der Dichte, der Geschwindigkeit von akustischen Wellen und der Absorption von akustischen Wellen in der Knochen-Barriere ausgehend von dem dreidimensionalen Bild,

(1c) Bestimmen einer vorbestimmten Position der Anordnung (17) von Transduktoren relativ zu der Knochen-Barriere (14),

(1d) Simulieren wenigstens einer Ausbreitung von akustischen Wellen zwischen wenigstens einem Punkt des im Wesentlichen homogenen Mediums und wenigstens bestimmten Transduktoren der Anordnung von Transduktoren, ausgehend von einem mathematischen Ausbreitungs-Modell und der dreidimensionalen Karten der Dichte, der Geschwindigkeit von akustischen Wellen und der Absorption von akustischen Wellen,

(1 e) Berechnen von akustischen Grundsignalen, welche von wenigstens bestimmten Transduktoren der Anordnung von Transduktoren zu emittieren sind, um das Soll-Feld von akustischen Wellen zu erhalten, auf Grundlage der Simulation,

- und eine reale Positionierungs-Phase der Anordnung von Transduktoren an der Knochen-Barriere, umfassend die Schritte:

(2a) zunächst näherungsweises Positionieren der Anordnung (17) von Transduktoren an der Knochen-Barriere (14) zunächst in ihrer vorgesehenen Position,

(2b) Verwenden wenigstens bestimmter Transduktoren der Anordnung von Transduktoren zum Bestimmen einer relativen Position zwischen der Anordnung (17) von Transduktoren und der Knochen-Barriere (14) mittels Echographie,

(2c) und Verbessern der Position der Anordnung (17) von Transduktoren relativ zu der Knochen-Barriere als eine Funktion der in Schritt (2b) durchgeführten Bestimmung.

2. Verfahren nach Anspruch 1, wobei während Schritt (2b) die relative Position zwischen der Anordnung (17) von Transduktoren und der Knochen-Barriere (14) durch Bestimmen einer äußeren Form wenigstens eines Teils der Knochen-Barriere durch Echographie bestimmt wird, unter Verwendung wenigstens eines Teils der Anordnung von Transduktoren, durch Vergleichen dieser äußeren Form mit dem dreidimensionalen Bild der Knochen-Barriere.

3. Verfahren nach Anspruch 1, wobei:

- dem Schritt (1a) ein anfänglicher Schritt vorausgeht, während welchem eine Positionsbestimmungs-Vorrichtung (8) an der Knochen-Barriere (14) fest angebracht wird, wobei die Positionionsbestimmungs-Vorrichtung (8) dazu vorgesehen ist, Röntgen-Strahlen zu absorbieren,

- während des Schritts (1a) aus dem aufgenommenen dreidimensionalen Bild ebenfalls eine Position der Positionsbestimmungs-Vorrichtung (8) auf der Knochen-Barriere (14) erhalten wird,

- und während des Schritts (2b) mittels Echographie eine relative Position zwischen der Anordnung (17) von Transduktoren und der Positionsbestimmungs-Vorrichtung (8) bestimmt wird.

4. Verfahren nach einem der vorhergehenden Ansprüche, in welchem die Anordnung von Transduktoren in einem flüssigkeitsgefüllten Behälter (13) aufgenommen ist, welcher wenigstens eine biegsame Wand (12) umfasst, und wobei während der Positionierungs-Phase die biegsame Wand in Anlage gegen die Knochen-Barriere (14) platziert wird.

5. Verfahren nach einem der vorhergehenden Ansprüche, wobei das im wesentlichen homogenen Medium (19) wenigstens einen Teil eines von einer Knochen-Barriere (14) verdeckten Gehirns umfasst, welche Knochen-Barriere wenigstens einen Teil eines das Gehirn umgebenden Schädels umfasst.

6. Verfahren nach einem der vorhergehenden Ansprüche, wobei die akustischen Wellen Frequenzen aufweisen, welche zwischen 0,5 und 3 MHz liegen.

7. Verfahren nach einem der vorhergehenden Ansprüche, wobei dem Schritt (1e) ein Schritt (1f) nachfolgt, während welchem wenigstens eine Emission von akustischen Signalen durch die Anordnung (17) von Transduktoren simuliert wird, welche Signale ausgehend von den akustischen Grundsignalen bestimmt werden und es erlauben, ein gewünschtes Feld aus akustischen Wellen zu erhalten, eine Ausbreitung der durch diese Emission erzeugten akustischen Wellen simuliert wird, und verifiziert wird, dass diese Ausbreitung bestimmte vordefinierte Kriterien erfüllt.

**8.** Verfahren nach einem der vorhergehenden Ansprüche, wobei:

- während des Schritts (1d) eine Ausbreitung von akustischen Wellen von wenigstens einem Punkt (18) des im Wesentlichen homogenen Mediums in Richtung wenigstens bestimmter Transduktoren der Anordnung von Transduktoren simuliert wird, und simulierte empfangene akustische Signale Ri(t) bestimmt werden, welche an der Position der Transduktoren i der Anordnung von Transduktoren ankommen, wobei i eine Ganzzahl zwischen 1 und n ist, und n die Anzahl der Transduktoren der Anordnung von Transduktoren ist,
- und während des Schritts (1e) die von jedem betrachteten Transduktor i zu emittierenden akustischen Grundsignale Ei(t) so bestimmt werden, dass sie proportional bezüglich einer Zeitinversion Ri(-t) der simulierten empfangenen akustischen Signale Ri(t) sind, welche zuvor in dem Schritt (1d) bestimmt worden sind.

**9.** Verfahren nach Anspruch 8, wobei während des Schritts (1e) die zu emittierenden akustischen Signale Ei(t) mittels der Formel Ei(t) = Gi*Ri(-t) bestimmt werden, wobei Gi ein Verstärkungsfaktor ist, welcher von einem Transduktor i zu dem anderen verschieden ist, um Verluste in der Knochen-Barriere zu kompensieren.

**10.** Verfahren nach Anspruch 9, wobei wenigstens bestimmten Transduktoren entsprechende Verstärkungsfaktoren Gi jeweils umgekehrt proportional zum Quadrat einer Amplitude der entsprechenden simulierten empfangenen akustischen Signale Ri(t) sind.

**11.** Verfahren nach Anspruch 8, wobei

- während des Schritts (1d) die Simulation unter Verwendung einer virtuellen dreidimensionalen Absorptions-Karte von akustischen Wellen durchgeführt wird, welche in jedem Punkt der Knochen-Barriere Absorptions-Koeffizienten $-\tau$ aufweist, welche den realen Absorptions-Koeffizienten $\tau$ entgegengesetzt sind, welche in Schritt (1b) bestimmt worden sind,
- und während Schritt (1e) die zu emittierenden akustischen Grundsignale Ei(t) so bestimmt werden, dass sie gleich der Zeitinversion Ri(-t) sind.

**12.** Verfahren nach einem der Ansprüche 1 bis 7, wobei die Anordnung (17) von Transduktoren in einem flüssigkeitsgefüllten Behälter (13) aufgenommen ist, welcher wenigstens eine biegsame Wand (12) umfasst, welche dazu vorgesehen ist, in Anlage gegen die Knochen-Barriere (14) platziert zu werden, wobei die während Schritt (1c) bestimmten, für die Transduktoren vorgesehenen Positionen nicht mit der Knochen-Barriere in Kontakt stehen, und wobei:

während des Schritts (1d):

- eine Ausbreitung von akustischen Wellen von wenigstens einem Punkt (18) des im Wesentlichen homogenen Mediums (19) in Richtung wenigstens bestimmter Transduktoren der Anordnung (17) von Transduktoren simuliert werden, und simulierte empfangene akustische Signale Ri(t) bestimmt werden, welche an der Position dieser Transduktoren i der Anordnung von Transduktoren ankommen, wobei i eine Ganzzahl zwischen 1 und n ist, und n die Anzahl der Transduktoren der Anordnung von Transduktoren ist,
- dann eine Emission eines akustischen Signals Ri(-t) von jedem Transduktor i, welches einer Zeitinversion des Signals Ri(t) entspricht, und eine Ausbreitung in der Flüssigkeit bis zu einem virtuellen Transduktor i simuliert wird, welcher in Kontakt mit der Knochen-Barriere (14) entsprechend dem Transduktor i positioniert ist, und simulierte empfangene akustische Signale R'i(t) bestimmt werden, welche an der Position des virtuellen Transduktors i ankommen,
- dann eine Emission eines akustischen Signals G'i*r'i(-t) von jedem virtuellen Transduktor i simuliert wird, wobei R'i(-t) eine Zeitinversion des Signals R'i(t) ist, und G'i ein Koeffizient ist, welcher wenigstens für bestimmte virtuelle Transduktoren i umgekehrt proportional zum Quadrat einer Amplitude des Signals R'i(t) ist,
- dann eine Ausbreitung in der Flüssigkeit bis zu dem Transduktor i simuliert wird, und simulierte empfangene akustische Signale R"i(t) bestimmt werden, welche an der Position i dieses Transduktors i ankommen,

und während des Schritts (1e) zu emittierende akustische Grundsignale Ei(t) so bestimmt werden, dass sie gleich einer Zeitinversion R"(-t) der simulierten empfangenen akustischen Signale R"i(t) sind.

**13.** Verfahren nach einem der Ansprüche 1 bis 7, wobei während des Schritts (1d) die Emission eines akustischen Wellenpulses von wenigstens bestimmten Transduktoren i der Anordnung (17) von Transduktoren und eine Ausbreitung von akustischen Wellen von jedem betrachteten Transduktor i in Richtung mehrerer Referenzpunkte r simuliert wird, welche in dem im Wesentlichen homogenen Medium (19) liegen, wobei i ein Index ist, der zwischen 1 und n liegt, welcher einen Transduktor der Anordnung bezeichnet, und n eine natürliche Ganzzahl ungleich Null ist, welche die Anzahl der Transduktoren bezeichnet, r eine Ganzzahl zwischen 1 um m ist, und m eine natürliche Ganzzahl ungleich Null ist, welche die Anzahl der Referenzpunkte bezeichnet, und simulierte Impuls-Antworten hri(t) bestimmt werden, welche an jedem der Referenzpunkte r des im Wesentlichen homogenen Mediums ankommen, wobei der Schritt (1e) die folgenden Unter-Schritte umfasst::

(1e1) Bestimmen einer Zahl p von Frequenz-Komponenten von jeder der simulierten Impuls-Antworten, von jeweiligen Frequenzen $\omega k$, wobei k ein Index zwischen 1 und p ist, welcher eine Frequenz-Komponente bezeichnet,

(1e2) Bestimmen von p Transfer-Matrizen $H(\omega k) = [Hri(\omega k)]$, wobei i von 1 bis n läuft und r von 1 bis m läuft, wobei $Hri(\omega k)$ der Wert der Fourier-Transformierten der Impuls-Antwort Hri(t) bei der Frequenz wk ist,

(1e3) Bestimmen von n Komponenten $Ei(\omega k,r)$ für jeden Referenzpunkt r, so dass $F(\omega k,r)=H(\omega k)*E(\omega k,r)$, wobei $E(\omega k,r)=[Ei(\omega k,r)]$ ein Vektor mit n Komponenten $Ei(\omega k,r)$ ist, $F(\omega k,r)=[Fl(\omega k,r)]$ ein Vektor mit m Komponenten $Fl(\omega k,r)$ ist, wobei l von 1 bis m variiert, wobei diese m Komponenten $Fl(\omega k, r)$ der Erzeugung des vorbestimmten Soll-Felds von akustischen Wellen mit der Frequenz $\omega k$ in den Punkten r entsprechen.

14. Verfahren nach Anspruch 13, wobei während des Unter-Schritts (1e3) p Matrizen $H^{-1}(\omega k)$ wenigstens durch Inversion der Transfer-Matrizen $H(\omega k)$ berechnet werden, und für jeden Referenzpunkt r des im Wesentlichen homogenen Mediums der Vektor $E(\omega k,r)$ mittels der Formel $E(\omega k,r) = H^{-1}(\omega k) * F(\omega k, r)$ berechnet wird.

15. Verfahren nach einem der vorhergehenden Ansprüche, wobei während des Schritts (1 d) Impuls-Antworten hir(t) zwischen mehreren Referenzpunkten r des im Wesentlichen homogenen Mediums und wenigstens bestimmten Transduktoren i der Anordnung (17) von Transduktoren bestimmt werden, wobei i ein Index zwischen 1 und n ist, welcher einen Transduktor bezeichnet, und n eine natürliche Ganzzahl ungleich Null ist, welche die Anzahl der Transduktoren bezeichnet, r eine Ganzzahl zwischen 1 und m ist und m eine natürliche Ganzzahl ungleich Null ist, welche die Anzahl der Referenzpunkte bezeichnet, und während des Schritts (1e) ebenfalls bestimmt wird, wie wenigstens ein Teil der Anordnung (17) von Transduktoren in einer Aufnahme in jedem Referenzpunkt r zu fokussieren ist, um ein echographisches Bild zu erhalten.

16. Verfahren zur medizinischen Bildaufnahme durch Echographie, umfassend ein nicht-invasives Verfahren um Erhalten eines Soll-Feldes von akustischen Wellen nach einem der vorhergehenden Ansprüche, und eine Bildaufnahme-Phase, während welcher wenigstens ein echographisches Bild des im Wesentlichen homogenen Mediums (19) unter Zuhilfenahme wenigstens eines Teils der Anordnung (17) von Transduktoren aufgenommen wird, unter Verwendung von akustischen Grundsignalen, welche während der Lernphase bestimmt werden.

17. Vorrichtung, welche insbesondere dazu vorgesehen ist, ein Verfahren nach einem der vorhergehenden Ansprüche durchzuführen, wobei die Vorrichtung wenigstens umfasst:

- eine Anordnung (17) von Transduktoren, welche dazu eingerichtet ist, an der Außenseite einer Knochen-Barriere (14) positioniert zu werden, welche ein im Wesentlichen homogenes Medium (19) verdeckt,
- Mittel (7) zum Bestimmen von wenigstens dreidimensionalen Karten der Dichte, der Geschwindigkeit von akustischen Wellen und der Absorption von akustischen Wellen in der Knochen-Barriere, ausgehend von einem dreidimensionalen Bild der Knochen-Barriere, mittels Röntgen-Strahlen, wobei die Porosität der Knochen-Barriere in jedem Punkt erhalten wird,
- Mittel (7) zum Simulieren wenigstens einer Ausbreitung von akustischen Wellen zwischen wenigstens einem Punkt (18) des im Wesentlichen homogenen Mediums und wenigstens bestimmten Transduktoren der Anordnung (17) von Transduktoren, ausgehend von einem mathematischen Ausbreitungs-Modell und den dreidimensionalen Karten der Dichte, der Geschwindigkeit von akustischen Wellen und der Absorption von akustischen Wellen, und als Funktion einer vorgesehenen Position der Anordnung (17) von Transduktoren relativ zu der Knochen-Barriere (14),
- Mittel (7) zum Berechnen von von wenigstens bestimmten Transduktoren der Anordnung (17) von Transduktoren zu emittierenden akustischen Grundsignalen, ausgehend von der Simulation, um ein Soll-Feld von akustischen Wellen in dem im Wesentlichen homogenen Medium zu erhalten,
- Mittel (7) zum Bestimmen einer relativen Position zwischen der Anordnung (17) von Transduktoren und der Knochen-Barriere (14) mittels Echographie durch wenigstens bestimmte Transduktoren der Anordnung (17) von Transduktoren,

- und Mittel (7, 4) zum Verbessern einer relativen AusgangsPosition der Anordnung (17) von Transduktoren relativ zu der Knochen-Barriere (14) als Funktion der Bestimmung der relativen Position zwischen der Anordnung von Transduktoren und der Knochen-Barriere, derart, dass die relative Position der Anordnung von Transduktoren relativ zu der Knochen-Barriere der vorgesehenen Position entspricht.

18. Vorrichtung nach Anspruch 17, in welcher die Mittel zum Verbessern der relativen Position zwischen der Anordnung (17) von Transduktoren und der Knochen-Barriere (14) dazu eingerichtet sind, eine äußere Form von wenigstens einem Teil der Knochen-Barriere (14) durch Echographie zu bestimmen, unter Zwischenverwendung wenigstens eines Teils der Anordnung (17) von Transduktoren, indem diese äußere Form mit dem dreidimensionalen Bild der Knochen-Barriere verglichen wird.

19. Vorrichtung nach Anspruch 17 oder Anspruch 18, umfassend eine mit Befestigungsmitteln (16) ausgestattete Positions-Bestimmungsvorrichtung (8), welche Befestigungsmittel dazu eingerichtet sind, die Positions-Bestimmungsvorrichtung an der Knochen-Barriere (14) zu befestigen, und wobei die Positions-Bestimmungsvorrichtung dazu eingerichtet ist, Röntgen-Strahlen zu absorbieren und auf dem dreidimensionalen Bild der Knochen-Barriere sichtbar zu sein, und wobei die Mittel (7) zum Bestimmen der relativen Position zwischen der Anordnung (17) von Transduktoren und der Knochen-Barriere (14) dazu eingerichtet sind, mittels Echographie eine relative Position zwischen der Anordnung (17) von Transduktoren und der Positions-Bestimmungsvorrichtung (8) zu bestimmen.

20. Vorrichtung nach einem der Ansprüche 17 bis 19, wobei die Anordnung (17) von Transduktoren in einem flüssigkeitsgefüllten Behälter (13) aufgenommen ist, welcher wenigstens eine biegsame Wand (12) umfasst, welche dazu vorgesehen ist, gegen die Knochen-Barriere (14) angelegt zu werden.

21. Vorrichtung nach einem der Ansprüche 17 bis 20, wobei die Anordnung (17) von Transduktoren eine Unter-Anordnung zur Hypothermie-Behandlung (17a) und eine Unter-Anordnung zur Bildaufnahme (17b) umfasst, wobei die beiden Unter-Anordnungen jeweils Transduktoren von verschiedenen Typen umfassen.

22. Vorrichtung nach einem der Ansprüche 17 bis 21, wobei die Anordnung (17) von Transduktoren dazu eingerichtet ist, akustische Wellen mit Frequenzen zu emittieren, welche zwischen 0,5 und 3 MHz liegen.

23. Vorrichtung nach einem der Ansprüche 17 bis 22, ferner umfassend Simulationsmittel (7), welche dazu eingerichtet sind:

- wenigstens eine Emission von akustischen Signalen durch die Anordnung (17) von Transduktoren zu simulieren, welche Signale ausgehend von den akustischen Grundsignale bestimmt sind und es erlauben, ein gewünschtes Feld von akustischen Wellen zu erhalten,
- Simulieren einer Ausbreitung von durch diese Emission erzeugten akustischen Wellen,
- und Verifizieren, dass diese Ausbreitung bestimmte vordefinierte Kriterien erfüllt.

24. Vorrichtung nach einem der Ansprüche 17 bis 23, wobei:

die Simulationsmittel (7) dazu eingerichtet sind, eine Ausbreitung von akustischen Wellen von wenigstens einem Punkt (18) des im Wesentlichen homogenen Mediums in Richtung wenigstens bestimmter Transduktoren der Anordnung von Transduktoren zu simulieren, um simulierte empfangene akustische Signale $R_i(t)$ zu bestimmen, welche an der Position der Transduktoren i der Anordnung von Transduktoren ankommen, wobei i eine Ganzzahl zwischen 1 und n ist, und n die Anzahl von Transduktoren der Anordnung von Transduktoren ist, und die Berechnungsmittel (7) dazu eingerichtet sind, die von jedem betrachteten Transduktor i zu emittierenden akustischen Grundsignale $E_i(t)$ als proportional zu einer Zeitinversion $R_i(-t)$ der simulierten empfangenen akustischen Signale zu bestimmen, welche zuvor in Schritt (1d) bestimmt worden sind.

25. Vorrichtung nach Anspruch 24, wobei die Berechnungsmittel (7) dazu eingerichtet sind, die zu emittierenden akustischen Signale $E_i(t)$ mittels der Formel $E_i(t) = G_i * R_i(-t)$ zu bestimmen, wobei $G_i$ ein Verstärkungsfaktor ist, welcher sich von einem Transistor i zu einem anderen unterscheidet, um Verluste in der Knochen-Barriere zu kompensieren.

26. Vorrichtung nach Anspruch 25, wobei die wenigstens bestimmten Transduktoren entsprechenden Verstärkungsfaktoren $G_i$ jeweils umgekehrt proportional zum Quadrat einer Amplitude der simulierten empfangenen akustischen Signale $R_i(t)$ sind.

**27.** Vorrichtung nach Anspruch 24, wobei
die Simulationsmittel (7) dazu eingerichtet sind, die Simulation der Ausbreitung von akustischen Wellen unter Verwendung einer virtuellen dreidimensionalen Karte einer Absorption von akustischen Wellen durchzuführen, welche in jedem Punkt der Knochen-Barriere Absorptions-Koeffizienten $-\tau$ aufweist, welche den realen Absorptions-Koeffizienten $\tau$ entgegengesetzt sind, welche während Schritt (1 b) bestimmt worden sind,
und die Berechnungsmittel (7) dazu eingerichtet sind, die zu emittierenden akustischen Grundsignale Ei(t) so zu bestimmen, dass sie gleich der Zeitinversion Ri(-t) sind.

**28.** Vorrichtung nach einem der Ansprüche 17 bis 23, wobei die Anordnung (17) von Transduktoren in einem flüssigkeitsgefüllten Behälter (13) aufgenommen ist, welcher wenigstens eine biegsame Wand (12) umfasst, welche dazu vorgesehen ist, in Anlage gegen die Knochen-Barriere (14) platziert zu werden, wobei die vorgesehenen Positionen der Transduktoren, welche von den Simulationsmitteln (7) berücksichtigt werden, nicht im Kontakt mit der Knochen-Barriere sind,
wobei die Simulationsmittel (7) dazu eingerichtet sind:

- eine Ausbreitung von akustischen Wellen von wenigstens einem Punkt (18) des im Wesentlichen homogenen Mediums (19) in Richtung wenigstens bestimmter Transduktoren der Anordnung (17) von Transduktoren zu stimulieren und simulierte empfangene akustische Signale Ri(t) zu bestimmen, welche an der Position der Transduktoren i der Anordnung von Transduktoren ankommen, wobei i eine Ganzzahl zwischen 1 und n ist und n die Anzahl von Transduktoren der Anordnung von Transduktoren ist,
- eine Emission eines akustischen Signals Ri(-t), welches einer Zeitinversion des Signals Ri(t) entspricht, von jedem Transduktor i und eine Ausbreitung in der Flüssigkeit bis zu einem virtuellen Transduktor i zu simulieren, welcher in Kontakt mit der Knochen-Barriere (14) entsprechend dem Transduktor i angeordnet ist, und simulierte empfangene akustische Signale R'i(t) zu bestimmen, welche an der Position des virtuellen Transduktors i ankommen,
- und eine Ausbreitung in der Flüssigkeit bis zu dem Transduktor i zu simulieren und simulierte empfangene akustische Signale R"i(t) zu bestimmen, welche an der Position des Transduktors i ankommen,

und wobei die Berechnungsmittel (7) dazu eingerichtet sind, die zu emittierenden akustischen Grundsignale Ei(t) so zu bestimmen, dass sie gleich einer Zeitinversion R"(-t) der simulierten empfangenen akustischen Signale R"i(t) sind.

**29.** Vorrichtung nach einem der Ansprüche 17 bis 23, wobei die Simulationsmittel (7) dazu eingerichtet sind:

- die Emission eines akustischen Wellen-Impulses von wenigstens bestimmten Transduktoren i der Anordnung (17) von Transduktoren und eine Ausbreitung von akustischen Wellen von jedem betrachteten Transduktor i in Richtung mehrerer Referenzpunkte r zu simulieren, welche in dem im Wesentlichen homogenen Medium (19) liegen, wobei i ein Index ist, welcher zwischen 1 und n liegt und n einen Transduktor der Anordnung bezeichnet, und n eine natürliche Ganzzahl ungleich Null ist, welche die Anzahl der Transduktoren bezeichnet, wobei r eine Ganzzahl ist, welche zwischen 1 und m liegt, und m eine natürliche Ganzzahl ungleich Null ist, welche die Anzahl der Referenzpunkte bezeichnet,
- die simulierten Impuls-Antworten hri(t) zu bestimmen, welche in jedem der Referenzpunkte r des im Wesentlichen homogenen Mediums ankommen,

und wobei die Berechnungsmittel (7) dazu eingerichtet sind:

- eine Zahl p von Frequenz-Komponenten von jedem der simulierten Impuls-Antworten bei jeweiligen Frequenzen $\omega k$ zu bestimmen, wobei k ein Index zwischen 1 und p ist, welcher eine FrequenzKomponente bezeichnet,
- p Transfer-Matrizen H($\omega k$)=[Hri($\omega k$)] zu bestimmen, wobei i von 1 bis n läuft und r von 1 bis m läuft, wobei Hri($\omega k$) der Wert der Fourier-Transformierten der Impuls-Antwort Hri(t) bei der Frequenz $\omega k$ ist,
- und Bestimmen von n Komponenten Ei($\omega k$,r) für jeden Referenzpunkt r, so dass F($\omega k$,r)=H($\omega k$)*E($\omega k$,r), wobei E($\omega k$,r)=[Ei($\omega k$,r)] ein Vektor mit n Komponenten Ei($\omega k$,r) ist, F($\omega k$,r)=[Fl($\omega k$,r)] ein Vektor mit m Komponenten Fl($\omega k$,r) ist, wobei l zwischen 1 und m variiert, wobei diese m Komponenten Fl($\omega k$,r) der Erzeugung des vorbestimmten Soll-Feldes von akustischen Wellen bei der Frequenz $\omega k$ an den Punkten r entsprechen.

**30.** Vorrichtung nach Anspruch 29, wobei die Berechnungsmittel (7) dazu eingerichtet sind:

- p Matrizen $H^{-1}(\omega k)$ wenigstens durch Inversion der Transfer-Matrizen H($\omega k$) zu berechnen,

- und für jeden Referenzpunkt r des im Wesentlichen homogenen Mediums den Vektor E(ωk,r) durch die Formel E(ωk,r)=H$^1$(ωk)*F(ωk,r) zu berechnen.

**31.** Vorrichtung nach einem der Ansprüche 17 bis 30, wobei die Simulationsmittel (7) dazu eingerichtet sind:

Impuls-Antworten hir(t) zwischen mehreren Referenzpunkten r des im Wesentlichen homogenen Mediums und wenigstens bestimmten Transduktoren i der Anordnung (17) von Transduktoren zu bestimmen, wobei i ein Index zwischen 1 und n ist, welcher einen Transduktor bezeichnet und n eine natürliche Ganzzahl ungleich Null ist, welche die Anzahl der Transduktoren bezeichnet, r eine Ganzzahl zwischen 1 und m ist und m eine natürliche Ganzzahl ungleich Null ist, welche die Anzahl der Referenzpunkte bezeichnet, und wobei die Berechnungsmittel (7) dazu eingerichtet sind, zu bestimmen, wie wenigstens ein Teil der Anordnung (17) von Transduktoren in einer Aufnahme in jedem Referenzpunkt r zu fokussieren ist, um ein echographisches Bild zu erhalten.

**32.** Vorrichtung nach einem der Ansprüche 17 bis 31, ferner umfassend Bildaufnahme-Mittel (7, 7b), welche dazu eingerichtet sind, wenigstens ein echographisches Bild des im Wesentlichen homogenen Mediums (19) unter Zuhilfenahme wenigstens eines Teils der Anordnung (17) von Transduktoren aufzunehmen, unter Verwendung der akustischen Grundsignale, welche von den Berechnungsmitteln (7) bestimmt worden sind.

**Claims**

**1.** A non-invasive method of obtaining at least one predetermined target soundwave field in a substantially homogeneous medium (19) masked by a bone barrier (14) by causing sound signals to be emitted by at least one array (17) of transducers through said bone barrier (14), the method being **characterized in that** it comprises:

· a training stage comprising at least the following steps:

1a) making a three-dimensional image of the bone barrier (14), at least in part by using X-rays, thereby obtaining a parameter representative of the porosity of said bone barrier at various points;
1b) from said three-dimensional image, determining three-dimensional maps of at least density, soundwave speed, and soundwave absorption in said bone barrier;
1c) determining a specific position for the array (17) of transducers relative to the bone barrier (14);
1d) simulating at least one propagation of soundwaves between at least one point of the substantially homogeneous medium and at least some of the transducers of the array of transducers on the basis of a mathematical model of propagation and said three-dimensional maps of density, soundwave speed, and soundwave absorption; and
1e) on the basis of said simulation, calculating individual sound signals to be emitted by at least some of the transducers of said array of transducers in order to obtain said target soundwave field;

· and a stage of actually positioning the array of transducers on the brain barrier, this stage comprising the following steps:

2a) initially positioning the array (17) of transducers on the bone barrier (14) approximately in the specific position;
2b) using at least some of the transducers of the array of transducers to perform echography to locate the position of said array (17) of transducers relative to the bone barrier (14); and
2c) refining the position of the array (17) of transducers relative to said bone barrier as a function of the location determined in step 2b).

**2.** A method according to claim 1, in which, during step 2b), the relative position between said array (17) of transducers and the bone barrier (14) is located by determining an outside shape of at least a portion of the bone barrier by echography using at least a portion of said array of transducers, and comparing said outside shape with said three-dimensional image of the bone barrier.

**3.** A method according to claim 1, in which:

· in step 1a), an initial step is performed during which a locating device (8) is rigidly secured on said bone barrier (14), said locating device (8) being adapted to absorb X-rays;

· during step 1a), the three-dimensional image made also gives the position of the locating device (8) on the bone barrier (14); and

· during step 2b), the relative position between the array (17) of transducers and the locating device (8) is identified by echography.

4. A method according to any preceding claim, in which the array of transducers is included in a fluid-filled tank (13) having at least one flexible wall (12), and during the positioning stage, said flexible wall is pressed against the bone barrier (14).

5. A method according to any one of the preceding claims, wherein the substantially homogeneous medium (19) comprises at least a portion of a brain masked by a bone barrier (14) comprising at least a portion of a skull surrounding said brain.

6. A method according to any preceding claim, in which the soundwaves are at frequencies lying in the range 0.5 MHz to 3 MHz.

7. A method according to any preceding claim, in which step 1e) is followed by a step 1f) during which emission of sound signals by the array (17) of transducers is simulated, said signals being determined from said individual sound signals and serving to obtain a desired soundwave field, propagation of soundwaves generated by said emission is simulated, and it is verified that said propagation satisfies certain predefined criteria.

8. A method according to any preceding claim, in which:

• during step 1d), propagation of soundwaves from at least one point (18) in the substantially homogeneous medium towards at least some transducers of the array of transducers is simulated, and received simulated sound signals $R_i(t)$ reaching the locations of said transducers $i$ of the array of transducers are determined, where $i$ is an integer in the range 1 to $\underline{n}$, and $\underline{n}$ is the number of transducers in the array of transducers; and

• during step 1e), the individual sound signals $E_i(t)$ for emission by each transducer $i$ under consideration are determined as being proportional to a time reversal $R_i(-t)$ of said received simulated sound signals $R_i(t)$ as previously determined in step 1d).

9. A method according to claim 8, in which, during step 1e), the sound signals $E_i(t)$ to be emitted are determined by the formula:

$$E_i(t) = G_i . R_i(-t)$$

where $G_i$ is a gain factor that differs from one transducer $\underline{i}$ to another, for compensating dissipation in the bone barrier.

10. A method according to claim 9, in which the gain factors $G_i$ corresponding to at least some of the transducers are respectively inversely proportional to the square of an amplitude of the corresponding received simulated sound signals $R_i(t)$.

11. A method according to claim 8, in which:

· during step 1d), the simulation is performed by using a virtual three-dimensional map of soundwave absorption, having absorption coefficients $-\tau$ at each point of the bone barrier that are opposite to the real absorption coefficients $\tau$ determined during step 1b); and

· during step 1e), the individual sound signals $E_i(t)$ to be emitted are determined as being equal to said time reversal $R_i(-t)$.

12. A method according to any one of claims 1 to 7, in which the array (17) of transducers is included in a fluid-filled tank (13) having at least one flexible wall (12) for pressing against the bone barrier (14), the specific positions of the transducers as determined during step 1c) not being in contact with the bone barrier, and in which:

during step 1d) :

· propagation of soundwaves from at least one point (18) of the substantially uniform medium (19) towards at least some transducers of the array (17) of transducers is determined, and received simulated sound signals Ri(t) reaching the locations of said transducers i of the array of transducers are determined, where i is an integer in the range 1 to n, and where n is the number of transducers in the array of transducers;
· then emission by each transducer i of a sound signal Ri(-t) corresponding to a time reversal of the signal Ri(t) is simulated, and propagation in said fluid to a virtual transducer i situated in contact with the bone barrier (14) in correspondence with the transducer i is simulated, and received simulated sound signals R'i(t) reaching the location of said virtual transducer i are determined;
· then emission by each virtual transducer i of an acoustic signal G'i.R'i(-t) is simulated where R'i(-t) is a time reversal of the signal R'i(t) and where G'i is a reversal coefficient proportional to the square of an amplitude of the signal R'i(t), at least for some of the virtual transducers i;
· then propagation in said fluid to the transducer i is simulated and received simulated sound signals R"i(t) reaching the location of said transducer i are determined;
and during step 1e), the individual sound signals Ei(t) to be emitted are determined as being equal to a time reversal R"i(-t) of said received simulated sound signals R"i(t).

13. A method according to any one of claims 1 to 7, in which during step 1d), the emission of a soundwave pulse by at least some of the transducers i of the array (17) of transducers is simulated and propagation of soundwaves from each transducer i in consideration towards a plurality of reference points r situated in the substantially uniform medium (19) is simulated, where i is an index in the range 1 to n designating a transducer of the array, and where n is a non-zero natural integer designating the number of transducers, r being an integer in the range 1 to m, where m is a non-zero natural integer designating the number of reference points, and simulated impulse responses hri(t) reaching each of said reference points r of the substantially homogeneous medium are determined, step 1e) comprising the following substeps:

1e1) determining a number p of frequency components for each of the simulated impulse responses, having respective frequencies $\omega k$, where k is an index lying in the range 1 to p and designating a frequency component;
1e2) determining p transfer matrices $H(\omega k) = [Hri(\omega k)]$, i lying in the range 1 to n, and r going from 1 to m, where $Hri(\omega k)$ is the value at the frequency $\omega k$ of the Fourier transform of the impulse response Hri(t); and
1e3) for each reference point r, n components $Ei(\omega k,r)$ are determined such that:

$$F(\omega k, r) = H(\omega k).E(\omega k, r)$$

where $E(\omega k,r) = [Ei(\omega k,r)]$ is a vector having n components $Ei(\omega k,r)$, $F(\omega k,r) = [F\ell(\omega k,r)]$ is a vector having m components $F\ell(\omega k,r)$ where $\ell$ varies in the range 1 to m, these m components $F\ell(\omega k,r)$ corresponding to generating said predetermined target soundwave field at the frequency $\omega k$ at the points r.

14. A method according to claim 13, in which, during substep 1e3), p matrices $H^{-1}(\omega k)$ are calculated at least by inverting the transfer matrices $H(\omega k)$, and for each reference point r of the substantially homogeneous medium, the vector $E(\omega k,r)$ is calculated using the formula:

$$E(\omega k, r) = H^{-1}(\omega k).F(\omega k, r)$$

15. A method according to any preceding claim, in which, during step 1d), the impulse responses hir(t) between a plurality of reference points r of the substantially homogeneous medium and at least some of the transducers i of the array (17) of transducers are determined, where i is an index in the range 1 to n which designates a transducer, and where n is a non-zero natural integer which designates the number of transducers, r being an integer lying in the range 1 to m, m being a non-zero natural integer designating the number of reference points, and during step 1e), it is also determined how to focus at least a portion of the array (17) of transducers in reception on each reference point r in order to make an echographic image.

16. A method of medical imaging by echography, the method comprising a non-invasive method of obtaining a target soundwave field according to any preceding claim, and an imaging stage during which at least one echographic image of the substantially homogeneous medium (19) is made using at least a portion of the array (17) of transducers, using the individual sound signals as determined during the training stage.

17. Apparatus specifically designed for implementing a method according to any preceding claim, the apparatus comprising at least:

· an array (17) of transducers adapted to be positioned outside a bone barrier (14) masking a substantially homogeneous medium (19);
· mapper means (7) for determining three-dimensional maps of at least density, soundwave speed, and soundwave absorption in the bone barrier, on the basis of a three-dimensional image of said bone barrier made by X-rays and giving the porosity of said bone barrier at each point;
· simulator means (7) for simulating at least one propagation of soundwaves between at least one point (18) of the substantially homogeneous medium and at least some of the transducers of the array (17) of transducers on the basis of a mathematical model of propagation and on the basis of said three-dimensional maps of density, of soundwave speed, and of soundwave absorption, and as a function of a specific position for the array (17) of transducers relative to the bone barrier (14);
· calculator means (7) for responding to said simulation to calculate individual sound signals to be emitted by at least some of the transducers of said array (17) of transducers in order to obtain a target soundwave field in the substantially homogeneous medium;
· locator means (7) for using at least some of the transducers of the array (17) of transducers to locate the position of said array (17) of transducers and relative to the bone barrier (14) by echography; and
· position-refiner means (7, 4) for refining an initial position of the array (17) of transducers relative to the bone barrier (14) as a function of the position of the array of transducers as located relative to the bone barrier in such a manner that the position of the array of transducers relative to the bone barrier corresponds to the specific position.

18. Apparatus according to claim 17, in which the means for locating the position of said array (17) of transducers relative to the bone barrier (14) are adapted to determine an outside shape of at least a portion of the bone barrier (14) by echography, using at least a portion of said array (17) of transducers, by comparing said outside shape with said three-dimensional image of said bone barrier.

19. Apparatus according to claim 17 or claim 18, comprising a locating device (8) provided with securing means (16) adapted to secure said locating device rigidly on the bone barrier (14), said locating device being adapted to absorb X-rays and being visible in the three-dimensional image of said bone barrier, and the means (7) for locating the position of said array (17) of transducers relative to the bone barrier (14) are adapted to locate the position of the array (17) of transducers relative to the locating device (8) by echography.

20. Apparatus according to any one of claims 17 to 19, in which the array (17) of transducers is included in a fluid-filled tank (13) having at least one flexible wall (12) for pressing against the bone barrier (14).

21. Apparatus according to any one of claims 17 to 20, in which the array (17) of transducers comprises both a sub-array (17b) for imaging and a sub-array (17a) for hyperthermia treatment, these two sub-arrays comprising transducers of respective different types.

22. Apparatus according to any one of claims 17 to 21, in which the array (17) of transducers is adapted to emit soundwaves at frequencies lying in the range 0.5 MHz to 3 MHz.

23. Apparatus according to any one of claims 17 to 22, further comprising simulator means (7) adapted:

· to simulate at least the array (17) of transducers emitting sound signals determined from said individual sound signals and enabling a desired soundwave field to be obtained;
· to simulate propagation of soundwaves generated by said emission; and
· to verify that said propagation satisfies certain predefined criteria.

24. Apparatus according to any one of claims 17 to 23, in which:

the simulator means (7) are adapted to simulate soundwave propagation from at least one point (18) of the substantially homogeneous medium towards at least some of the transducers of the array of transducers to determine received simulated sound signals Ri(t) reaching the locations of said transducers $\underline{i}$ of the array of transducers, $\underline{i}$ being an integer in the range 1 to $\underline{n}$, and $\underline{n}$ being the number of transducers in the array of transducers; and

the calculator means (7) are adapted to determine the individual sound signals Ei(t) to be emitted by each transducer $\underline{i}$ under consideration as being proportional to a time reversal Ri(-t) of said received simulated sound signals Ri(t) as previously determined in step 1d).

**25.** Apparatus according to claim 24, in which the calculator means (7) are adapted to determine the sound signals Ei(t) to be emitted using the formula:

$$\mathrm{Ei(t)\ =\ Gi.\overline{Ri}(-t)}$$

where Gi is a gain factor that differs from one transducer $\underline{i}$ to another, in order to compensate for dissipation in the bone barrier.

**26.** Apparatus according to claim 25, in which the gain factors Gi corresponding to at least some of the transducers are respectively inversely proportional to the square of an amplitude of the corresponding received simulated sound signals Ri(t).

**27.** Apparatus according to claim 24, in which:

the simulator means (7) are adapted to perform said simulation of soundwave propagation by using a virtual three-dimensional map of soundwave absorption having at each point of the bone barrier absorption coefficients of $-\tau$ opposite to the real absorption coefficient $\tau$ as determined during step 1b); and
the calculator means (7) are adapted to determine the individual sound signals Ei(t) to be emitted as being equal to said time reversal Ri(-t).

**28.** Apparatus according to any one of claims 17 to 23, in which the array (17) of transducers is included in a fluid-filled tank (13) having at least one flexible wall (12) for pressing against the bone barrier (14), the locations provided for the transducers taken into account by the simulator means (7) not being in contact with the bone barrier, in which apparatus the simulator means (7) are adapted:

· to simulate propagation of soundwaves from at least one point (18) of the substantially homogeneous medium (19) towards at least some of the transducers of the array (17) of transducers and to determine the received simulated sound signals Ri(t) reaching the locations of said transducers $\underline{i}$ of the array of transducers, $\underline{i}$ being an integer in the range 1 to n, and $\underline{n}$ being the number of transducers in the array of transducers;
· to simulate emission by each transducer $\underline{i}$ of a soundwave Ri(-t) corresponding to a time reversal of the signal Ri(t), and to simulate propagation in said fluid to a virtual transducer $\underline{i}$ situated in contact with the bone barrier (14) in correspondence with the transducer $\underline{i}$, and to determine the received simulated sound signals R'i(t) reaching the location of said virtual transducer $\underline{i}$;
· to simulate emission by each virtual transducer $\underline{i}$ of a sound signal G'i.R'i(-t) where R'i(-t) is a time reversal of the signal R'i(t), and where G'i is a reversal coefficient proportional to the square of an amplitude of the signal R'i(t), at least for some of the virtual transducers $\underline{i}$; and
· to simulate propagation in said fluid to the transducer $\underline{i}$, and to determine the received simulated sound signals R"i(t) reaching the location of said transducer $\underline{i}$;
and in which the calculator means (7) are adapted to determine the individual sound signals Ei(t) to be emitted as being equal to a time reversal R"i(-t) of said received simulated sound signals R"i(t).

**29.** Apparatus according to any one of claims 17 to 23, in which the simulator means (7) are adapted:

· to simulate emission of a soundwave pulse by at least some of the transducers $\underline{i}$ of the array (17) of transducers, and propagation of soundwaves from each transducer $\underline{i}$ in question towards a plurality of reference points $\underline{r}$ situated in the substantially homogeneous medium (19), i being an index in the range 1 to $\underline{n}$ designating a transducer of the array, and $\underline{n}$ being a non-zero natural integer designating the number of transducers, r being an integer in the range 1 to $\underline{m}$, and $\underline{m}$ being a non-zero natural integer designating the number of reference points;

· to determine simulated impulse responses hri(t) reaching each of said reference points $\underline{r}$ of the substantially homogeneous medium;

and in which the calculator means (7) are adapted:

· to determine a number $\underline{p}$ of frequency components for each of said simulated impulse responses at respective frequencies $\omega k$, $\underline{k}$ being an index in the range 1 to $\underline{p}$ and designating a frequency component;
· to determine $\underline{p}$ transfer matrices $H(\omega k) = [Hri(\omega k)]$, $\underline{i}$ lying in the range 1 to $\underline{n}$ and $\underline{r}$ lying in the range 1 to $\underline{m}$, where $Hri(\omega k)$ is the value at the frequency $\omega k$ of the Fourier transform of the impulse response $Hri(t)$; and
· to determine, for each reference point $\underline{r}$, $\underline{n}$ components $Ei(\omega k,r)$ such that:

$$F(\omega k, r) = H(\omega k) . E(\omega k, r)$$

where $E(\omega k,r) = [Ei(\omega k,r)]$ is a vector having $\underline{n}$ components $Ei(\omega k,r)$, $F(\omega k,r) = [F\ell(\omega k,r)]$ is a vector of $\underline{m}$ components $F\ell(\omega k,r)$ where $\ell$ lies in the range 1 to $\underline{m}$, these $\underline{m}$ components $F\ell(\omega k,r)$ corresponding to generating said predetermined target soundwave field at the frequency $\omega k$ at the points $\underline{r}$.

**30.** Apparatus according to claim 29, in which the calculator means (7) are adapted:

· to calculate $\underline{p}$ matrices $H^{-1}(\omega k)$ at least by inverting the transfer matrices of $H(\omega k)$; and
· for each reference point $\underline{r}$ of the substantially homogeneous medium, to calculate the vector $E(\omega k, r)$ by means of the formula:

$$E(\omega k, r) = H^{-1}(\omega k) . F(\omega k, r)$$

**31.** Apparatus according to any one of claims 17 to 30, in which the simulator means (7) are adapted:

· to determine the impulse responses hir(t) between a plurality of reference points $\underline{r}$ of the substantially homogeneous medium and at least some of the transducers $\underline{i}$ of the array (17) of transducers, $\underline{i}$ being an index lying in the range 1 to $\underline{n}$ designating a transducer, and $\underline{n}$ being a non-zero natural integer designating the number of transducers, $\underline{r}$ being an integer lying in the range 1 to $\underline{m}$, and $\underline{m}$ being a non-zero natural integer designating the number of reference points;
and in which the calculator means (7) are adapted to determine how to focus at least a portion of the array (17) of transducers in reception on each reference point $\underline{r}$ in order to make an echographic image.

**32.** Apparatus according to any one of claims 17 to 31, further comprising imaging means (7, 7b) adapted to make at least one echographic image of the substantially homogeneous medium (19) using at least a portion of the array (17) of transducers, using the individual sound signals determined by the calculator means (7).

EP 1 531 734 B1

FIG. 1

**FIG. 2**

**FIG. 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0232316 A

**Littérature non-brevet citée dans la description**

- **M. PERNOT et al.** Improvement of Ultrasound Based Temperature Estimation by Compound Imaging. *Proceedings of the International Symposium on Therapeutic Ultrasound, Seattle,* 2002

- **ENTREKIN et al.** Real Time Spatial Compound Imaging in breast ultrasound: technology and early clinical experience. *Medica Mundi,* Septembre 1999, vol. 43, 35-43